(19) Europäisches Patentamt
European Patent Office
Office européen des brevets

(11) **EP 4 264 272 B1**

(12) **EUROPEAN PATENT SPECIFICATION**

(45) Date of publication and mention of the grant of the patent:
**18.09.2024  Bulletin 2024/38**

(21) Application number: **21830712.2**

(22) Date of filing: **16.12.2021**

(51) International Patent Classification (IPC):
**G01N 33/68** *(2006.01)*    **G01N 33/92** *(2006.01)*

(52) Cooperative Patent Classification (CPC):
**G01N 33/6887; G01N 33/6812; G01N 33/92;**
G01N 2800/105; G01N 2800/50

(86) International application number:
**PCT/FI2021/050888**

(87) International publication number:
**WO 2022/129702 (23.06.2022 Gazette 2022/25)**

(54) **METHOD FOR DETERMINING WHETHER A SUBJECT IS AT RISK OF DEVELOPING A MUSCULOSKELETAL AND/OR CONNECTIVE TISSUE DISEASE**

VERFAHREN ZUR BESTIMMUNG DES RISIKOS EINER PERSON ZUR ENTWICKLUNG EINER MUSKELSKELETT- UND/ODER BINDEGEWEBEERKRANKUNG

MÉTHODE PERMETTANT DE DÉTERMINER SI UN SUJET PRÉSENTE UN RISQUE DE DÉVELOPPER UNE MALADIE MUSCULO-SQUELETTIQUE OU UNE MALADIE DES TISSUS CONJONCTIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2020  FI 20206343**

(43) Date of publication of application:
**25.10.2023  Bulletin 2023/43**

(73) Proprietor: **NIGHTINGALE HEALTH OYJ**
**00300 Helsinki (FI)**

(72) Inventors:
• **JULKUNEN, Heli**
**00300 Helsinki (FI)**
• **WÜRTZ, Peter**
**00300 Helsinki (FI)**

(74) Representative: **Papula Oy**
**P.O. Box 981**
**00101 Helsinki (FI)**

(56) References cited:
**WO-A1-2013/053065**

• **ZENGINI ELENI ET AL: "Genome-wide analyses using UK Biobank data provide insights into the genetic architecture of osteoarthritis", NATURE GENETICS, NATURE PUBLISHING GROUP US, NEW YORK, vol. 50, no. 4, 20 March 2018 (2018-03-20), pages 549 - 558, XP036928243, ISSN: 1061-4036, [retrieved on 20180320], DOI: 10.1038/S41588-018-0079-Y**
• **RITCHIE SCOTT C. ET AL: "The Biomarker GlycA Is Associated with Chronic Inflammation and Predicts Long-Term Risk of Severe Infection", CELL SYSTEMS, vol. 1, no. 4, 1 October 2015 (2015-10-01), US, pages 293 - 301, XP055837905, ISSN: 2405-4712, Retrieved from the Internet <URL:https://www.cell.com/cell-systems/pdf/S2405-4712(15)00145-3.pdf> DOI: 10.1016/j.cels.2015.09.007**

**Description**

**TECHNICAL FIELD**

The present disclosure relates generally to methods for determining whether a subject is at risk of developing a musculoskeletal and/or connective tissue disease.

**BACKGROUND**

Musculoskeletal and connective tissue diseases comprise disorders related to the locomotor system. These diseases often involve joints, bones, muscles and associated tissues, such as tendons and ligaments. Common disorders of the musculoskeletal system include, for instance, rheumatoid arthritis, osteoarthritis and spondylosis. These are often long-term or even chronic conditions that cause pain, swelling and stiffness, having a significant impact on the physical function, productivity and quality of life of the patients. There may also be periods where the symptoms become worse, and some people with these disorders also experience problems in other parts of the body and more general symptoms such as fatigue and weight loss as well as inflammation in the lungs and in the heart.

Identifying individuals at risk for future onset of musculoskeletal and connective tissue diseases and related symptoms is important to minimise and prevent long-term damage to the musculoskeletal system. Various blood biomarkers may be useful for predicting whether an individual person is at an elevated risk of developing a broad range musculoskeletal system and connective tissue diseases as well as specific types of these disorders, such as rheumatoid arthritis, other arthritis, gout, osteoarthritis, joint disorders, spondylosis and/or soft tissue disorders. Biomarkers predictive of the onset of these disorders could help to enable more effective screening and better targeted early treatment and prevention methods. Such biomarkers may be measured from biological samples, for example from blood samples or related biological fluids.

The international patent application WO 2013/053065 A1 discloses a method of determining whether a subject is at risk of developing osteoarthritis (OA) but among the biomarkers disclosed glycoprotein acetyls are not mentioned.

**SUMMARY**

The invention in its broadest form is defined in independent claim 1:

A method for determining whether a subject is at risk of developing a musculoskeletal disease;wherein the method comprises determining in a biological sample obtained from the subject a quantitative value of at least one biomarker of the following in the biological sample:- glycoprotein acetyls,- albumin,- a ratio of docosahexaenoic acid to total fatty acids,- a ratio of linoleic acid to total fatty acids- a ratio of monounsaturated fatty acids and/or of oleic acid to total fatty acids,- a ratio of omega-6 fatty acids to total fatty acids,- a ratio of saturated fatty acids to total fatty acids,- fatty acid degree of unsaturation,- docosahexaenoic acid,- linoleic acid,-monounsaturated fatty acids and/or oleic acid,- omega-6 fatty acids,-saturated fatty acids,- triglycerides in high-density lipoprotein (HDL),- triglycerides in low-density lipoprotein (LDL),- high-density lipoprotein (HDL) particle size,- low-density lipoprotein (LDL) particle size,- very-low-density lipoprotein (VLDL) particle size,-acetate,- citrate,- glutamine,- glycine,- histidine,- isoleucine,-leucine,- phenylalanine,- valine; and comparing the quantitative value(s) of the at least one biomarker to a control sample or to a control value;wherein an increase or a decrease in the quantitative value (s) of the at least one biomarker, when compared to the control sample or to the control value, is/are indicative of the subject having an increased risk of developing a musculoskeletal disease;wherein the at least one biomarker comprises or is glycoprotein acetyls; and wherein the musculoskeletal disease comprises or is osteoarthritis.

**BRIEF DESCRIPTION OF THE DRAWINGS**

The accompanying drawings, which are included to provide a further understanding of the embodiments and constitute a part of this specification, illustrate various embodiments. In the drawings:

Figure 1a shows the relation of baseline concentrations of 27 blood biomarkers to future development of Any Musculoskeletal and/or Connective Tissue Disease (defined as the combined endpoint of any ICD-10 diagnoses within M00-M99; here termed "Any Musculoskeletal and/or Connective Tissue Disease"), when the biomarker concentrations are analysed in absolute concentrations and in quintiles of biomarker concentrations. Results are based on plasma samples from approximately 115 000 generally healthy individuals from the UK Biobank.

Figure 1b shows the cumulative risk for "Any Musculoskeletal and/or Connective Tissue Disease" during follow-up for the lowest, middle, and highest quintiles of the 27 blood biomarker concentrations.

Figure 2a shows the relation of the baseline concentrations of the 27 blood biomarkers to future development of 7 different categories of musculoskeletal and/or connective tissue diseases (defined by ICD-10 subchapters), in the form of a heatmap. The results demonstrate that the 7 different musculoskeletal and/or connective tissue disease subgroups all have highly similar associations with the 27 biomarkers measured by nuclear magnetic resonance (NMR) spectroscopy of plasma samples from generally healthy humans.

**Figure 2b** shows the consistency of the biomarker associations with the 7 different categories of musculoskeletal and/or connective tissue diseases, defined by ICD-10 subchapters, in comparison to the direction of corresponding biomarker associations with "Any Musculoskeletal and/or Connective Tissue Disease".

**Figure 3a** shows the relation of baseline biomarker levels to the future development of 13 specific musculoskeletal and/or connective tissue diseases (defined by ICD-10 3-character diagnoses) in the form of a heatmap. The results demonstrate that the specific musculoskeletal and/or connective tissue diseases defined by 3-character ICD-10 codes all have highly similar associations with a broad panel of biomarkers measured by NMR spectroscopy of plasma samples from generally healthy humans.

**Figure 3b** shows the consistency of the biomarker associations with specific musculoskeletal and/or connective tissue diseases (defined by ICD-10 3-character diagnoses), in comparison to direction of the association with "Any Musculoskeletal and/or Connective Tissue Disease".

**Figures 4a-d** show the relation of baseline biomarker levels to the future development of 7 different musculoskeletal and/or connective tissue disease categories (defined by ICD-10 subchapters), in the form of forestplots of the hazard ratios for incident disease onset.

**Figures 5a-g** show the relation of baseline biomarker levels to the future development of 13 different musculoskeletal and/or connective tissue diseases (defined by ICD-10 3-character diagnoses), in the form of forestplots of the hazard ratios for incident disease onset.

**Figure 6** shows an example of the relation of multi-biomarker scores to the risk of "Any Musculoskeletal and/or Connective Tissue Disease". Selected examples of multi-biomarker scores are shown to illustrate the improved prediction attained by multi-biomarker scores as compared to individual biomarkers.

**Figure 7a** shows an intended use case for a multi-biomarker score to predict the risk for developing rheumatoid arthritis among initially healthy humans.

**Figure 7b** shows that the prediction of the risk for developing rheumatoid arthritis works effectively for people with different demographics and risk factor profiles.

**Figure 8a** shows an intended use case for a multi-biomarker score to predict the risk for developing gout among initially healthy humans.

**Figure 8b** shows that the prediction of the risk for developing gout works effectively for people with different demographics and risk factor profiles.

**Figure 9a** shows an intended use case for a multi-biomarker score to predict the risk for developing arthritis among initially healthy humans.

**Figure 9b** shows that the prediction of the risk for developing arthritis works effectively for people with different demographics and risk factor profiles.

**Figure 10a** shows an intended use case for a multi-biomarker score to predict the risk for developing osteoarthritis among initially healthy humans.

**Figure 10b** shows that the prediction of the risk for developing osteoarthritis works effectively for people with different demographics and risk factor profiles.

**Figure 11a** shows an intended use case for a multi-biomarker score to predict the risk for developing other joint disorder among initially healthy humans.

**Figure 11b** shows that the prediction of the risk for developing other joint disorder works effectively for people with different demographics and risk factor profiles.

**Figure 12a** shows an intended use case for a multi-biomarker score to predict the risk for developing spondylosis among initially healthy humans.

**Figure 12b** shows that the prediction of the risk for developing spondylosis works effectively for people with different demographics and risk factor profiles.

**Figure 13a** shows an intended use case for a multi-biomarker score to predict the risk for developing soft tissue disorders among initially healthy humans.

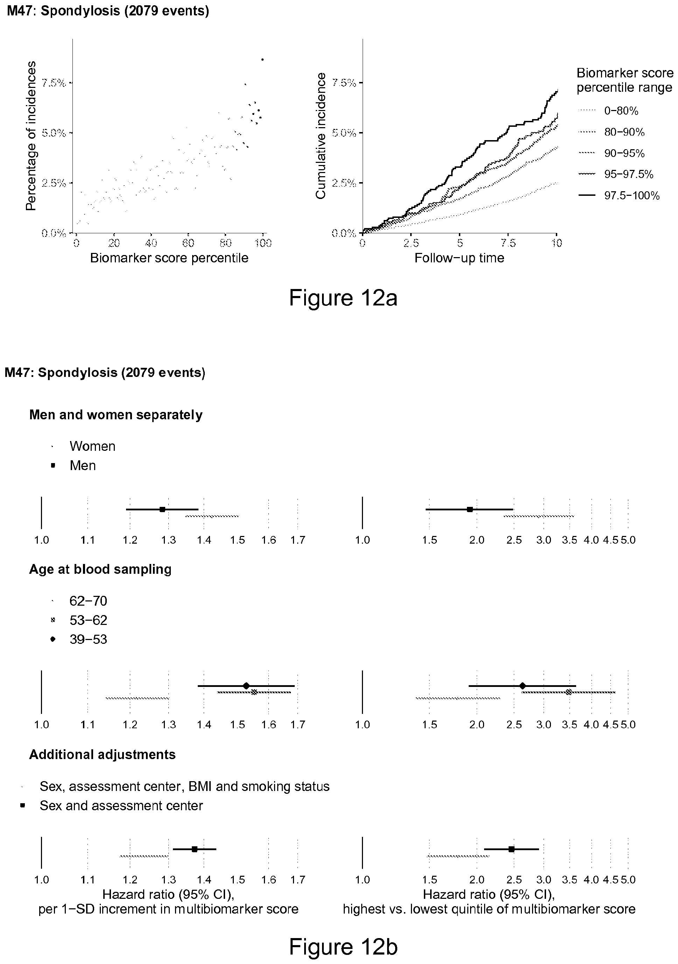

Figure 12b shows that the prediction of the risk for developing soft tissue disorders works effectively for people with different demographics and risk factor profiles.

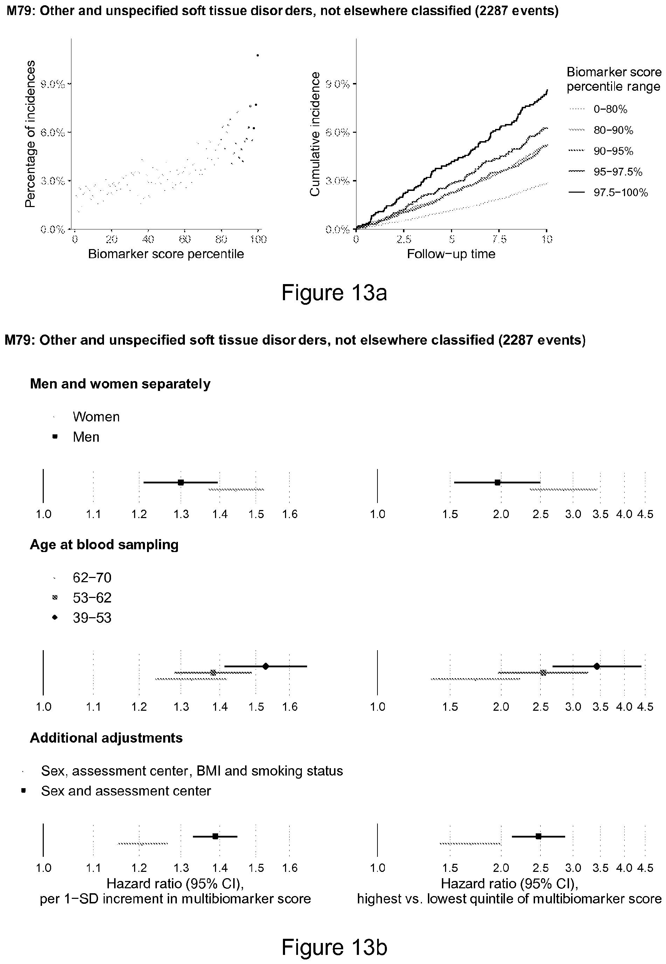

**Figure 13b** shows that the prediction of the risk for developing soft tissue disorders works effectively for people with different demographics and risk factor profiles.

## DETAILED DESCRIPTION

A method for determining whether a subject is at risk of developing a musculoskeletal and/or connective tissue disease is disclosed.

The musculoskeletal and/or connective tissue disease may be e.g. a disease of the musculoskeletal system or a disease of the connective tissue, such as rheumatoid arthritis, other arthritis, osteoarthritis, other joint disorder, spondylosis and/or other soft tissue disorder.

The method may comprise determining in a biological sample obtained from the subject a quantitative value of at least one biomarker of the following:

- albumin,
- glycoprotein acetyls,
- a ratio of docosahexaenoic acid to total fatty acids,
- a ratio of linoleic acid to total fatty acids
- a ratio of monounsaturated fatty acids and/or of oleic acid to total fatty acids,
- a ratio of omega-6 fatty acids to total fatty acids,
- a ratio of saturated fatty acids to total fatty acids,
- fatty acid degree of unsaturation,
- docosahexaenoic acid,
- linoleic acid,
- monounsaturated fatty acids and/or oleic acid,
- omega-6 fatty acids,
- saturated fatty acids,
- triglycerides in high-density lipoprotein (HDL),
- triglycerides in low-density lipoprotein (LDL),
- high-density lipoprotein (HDL) particle size,
- low-density lipoprotein (LDL) particle size,
- very-low-density lipoprotein (VLDL) particle size,
- acetate,
- citrate,
- glutamine,
- glycine,
- histidine,
- isoleucine,
- leucine,
- phenylalanine,
- valine; and

and comparing the quantitative value(s) of the at least one biomarker to a control sample or to a control value; wherein an increase or a decrease in the quantitative value (s) of the at least one biomarker, when compared to the control sample or to the control value, is/are indicative of the subject having an increased risk of developing the musculoskeletal and/or connective tissue disease.

Various biomarkers may be useful for predicting whether an individual person is at elevated risk of developing a broad range of musculoskeletal and/or connective tissue diseases. Such biomarkers may be measured from biological samples, for example from blood samples or related biological fluids.

Biomarkers predictive of hospitalization from a musculoskeletal and/or connective tissue disease could help to enable more effective screening and better targeted preventative treatment.

In an embodiment, the method is a method for determining whether a subject is at risk of being hospitalized from a musculoskeletal and/or connective tissue disease, and an increase or a decrease in the quantitative value (s) of the at least one biomarker, when compared to the control sample or to the control value, is/are indicative of the subject having an increased risk of being hospitalized from the musculoskeletal and/or connective tissue disease.

In an embodiment, the method comprises determining a quantitative value of albumin.

In an embodiment, the method comprises determining a quantitative value of glycoprotein acetyls.

In an embodiment, the method comprises determining a quantitative value of the ratio of docosahexaenoic acid to total fatty acids.

In an embodiment, the method comprises determining a quantitative value of the ratio of linoleic acid to total fatty acids.

In an embodiment, the method comprises determining a quantitative value of the ratio of monounsaturated fatty acids and/or oleic acid to total fatty acids.

In an embodiment, the method comprises determining a quantitative value of the ratio of omega-6 fatty acids to total fatty acids.

In an embodiment, the method comprises determining a quantitative value of the ratio of saturated fatty acids to total fatty acids.

In an embodiment, the method comprises determining a quantitative value of fatty acid degree of unsaturation.

In an embodiment, the method comprises determining a quantitative value of docosahexaenoic acid.

In an embodiment, the method comprises determining a quantitative value of linoleic acid.

In an embodiment, the method comprises determining a quantitative value of monounsaturated fatty acids and/or oleic acid.

In an embodiment, the method comprises determining a quantitative value of omega-6 fatty acids.

In an embodiment, the method comprises determining a quantitative value of saturated fatty acids.

In an embodiment, the method comprises determining a quantitative value of triglycerides in high-density lipoprotein (HDL) .

In an embodiment, the method comprises determining a quantitative value of triglycerides in low-density lipoprotein (LDL).

In an embodiment, the method comprises determining a quantitative value of high-density lipoprotein (HDL) particle size.

In an embodiment, the method comprises determining a quantitative value of low-density lipoprotein (LDL) particle size.

In an embodiment, the method comprises determining a quantitative value of very-low-density lipoprotein (VLDL) particle size.

In an embodiment, the method comprises determining a quantitative value of acetate.

In an embodiment, the method comprises determining a quantitative value of citrate.

In an embodiment, the method comprises determining a quantitative value of glutamine.

In an embodiment, the method comprises determining a quantitative value of glycine.

In an embodiment, the method comprises determining a quantitative value of histidine.

In an embodiment, the method comprises determining a quantitative value of isoleucine.

In an embodiment, the method comprises determining a quantitative value of leucine.

In an embodiment, the method comprises determining a quantitative value of phenylalanine.

In an embodiment, the method comprises determining a quantitative value of valine.

The metabolic biomarker(s) described in this specification have been found to be significantly different, i.e. their quantitative values (such as amount and/or concentration) have been found to be significantly higher or lower, for subjects who later developed a musculoskeletal and/or connective tissue disease. The biomarkers may be detected and quantified from blood, serum, or plasma, a dry blood spot, or other suitable biological sample, and may be used to determine the risk of developing a musculoskeletal and/or connective tissue disease, either alone or in combination with other biomarkers.

Furthermore, the biomarker(s) may significantly improve the possibility of identifying subjects at risk for a musculoskeletal and connective tissue disease, even in combination with and/or when accounting for established risk factors that may currently be used for screening and risk prediction, such as age, sex, smoking status, body mass index (BMI), occupational and ergonomic practices, inflammatory protein biomarkers such as C-reactive protein, family history, genetic risk and/or prior medical history of having musculoskeletal and/or connective tissue diseases and/or other comorbidities. The biomarkers described in this specification, alone or as a risk score (such as a multi-biomarker score), hazard ratio, odds ratio, and/or predicted absolute or relative risk, or in combination with other risk factors and tests, may improve prediction or even replace the need for other tests or measures. This may include improving prediction accuracy by complementing the predictive information from other risk factors, or by replacing the need for other analyses, such as blood test to identify rheumatoid factor or anti-cyclic citrullinated peptide (anti-CCP) antibody, arthroscopy, joint aspiration (arthrocentesis), nerve and muscle tests and/or medical imaging techniques such as arthrography, bone scanning, ultrasonography, computed tomography (CT) or magnetic resonance imaging (MRI). The biomarkers or the risk score, hazard ratio, odds ratio, and/or predicted absolute or relative risk according to one or more embodiments described in this specification may thus allow for efficiently assessing the risk for future development of a musculoskeletal and/or connective tissue disease, also in conditions in which other risk factor measures are not as feasible.

The method may comprise determining in the biological sample quantitative values of a plurality of the biomarkers, such as two, three, four, five or more biomarkers. For example, the plurality of the biomarkers may comprise 2, 3, 4, 5, 6, 7, 8, 9, 10, 11, 12, 13, 14, 15, 16, 17, 18, 19, 20, 21, 22, 23, 24, 25, 26 or 27 (i.e. at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26 or all of the biomarkers) of the biomarkers. The term "plurality of the biomarkers" may thus, within this specification, be understood as referring to any number (above one) of the biomarkers. The term "plurality of the biomarkers" may thus be understood as referring to any number (above one) and/or combination or subset of the biomarkers described in this specification. Determining the plurality of the biomarkers may increase the accuracy of the prediction of whether the subject is at risk of developing a musculoskeletal and/or connective tissue disease. In general, it may be that the higher the number of the biomarkers, the more accurate or predictive the method. However, even a single biomarker described in this specification may allow for or assist in determining whether the subject is at risk of developing a musculoskeletal and/or connective tissue disease. The plurality of the biomarkers may be measured from the same biological sample or from separate biological samples and using the same analytical method or different analytical methods. In an embodiment, the plurality of biomarkers may be a panel of a plurality of biomarkers.

In the context of this specification, the wording "comparing the quantitative value(s) of the biomarker(s) to a control sample or to a control value(s)" may be understood, as a skilled person would, as referring to comparing the quantitative value or values of the biomarker or biomarkers, to a control sample or to a control value(s) either individually or as a plurality of biomarkers (e.g. when a risk score is calculated from the quantitative values of a plurality of biomarkers), depending e.g. on whether the quantitative value of a single (individual) biomarker or the quantitative values of a plurality of biomarkers are determined.

In an embodiment, the method may comprise determining in the biological sample obtained from the subject a quantitative value of the following biomarkers:

- albumin,
- glycoprotein acetyls,
- a ratio of docosahexaenoic acid to total fatty acids,
- a ratio of linoleic acid to total fatty acids
- a ratio of monounsaturated fatty acids and/or of oleic acid to total fatty acids,
- a ratio of omega-6 fatty acids to total fatty acids,
- a ratio of saturated fatty acids to total fatty acids,
- fatty acid degree of unsaturation,
- docosahexaenoic acid,
- linoleic acid,
- monounsaturated fatty acids and/or oleic acid,
- omega-6 fatty acids,
- saturated fatty acids,
- triglycerides in high-density lipoprotein (HDL),
- triglycerides in low-density lipoprotein (LDL),
- high-density lipoprotein (HDL) particle size,
- low-density lipoprotein (LDL) particle size,
- very-low-density lipoprotein (VLDL) particle size,
- acetate,
- citrate,
- glutamine,
- glycine,
- histidine,
- isoleucine,
- leucine,
- phenylalanine,
- valine; and

comparing the quantitative value(s) of the biomarkers to a control sample or to a control value(s);
wherein an increase or a decrease in the quantitative value(s) of the biomarkers, when compared to the control sample or to the control value, is/are indicative of the subject having an increased risk of developing a musculoskeletal and/or connective tissue disease.

In an embodiment, the at least one biomarker comprises or is glycoprotein acetyls. The method may further comprise determining a quantitative value of at least one of the other biomarkers described in this specification.

The subject may be human. The human may be healthy or have an existing disease, such as an existing musculoskeletal and/or connective tissue disease. Specifically, the human may have an already existing form of a musculoskeletal and/or connective tissue disease, and the risk for developing more severe forms of this disease and/or other musculoskeletal and/or connective tissue diseases may be determined and/or calculated. The subject may, additionally or alternatively, be an animal, such as a mammal, for example, a non-human primate, a dog, a cat, a horse, or a rodent.

In the context of this specification, the term "biomarker" may refer to a biomarker, for example a chemical or molecular marker, that may be found to be associated with a disease or a condition or the risk of having or developing thereof. It does not necessarily refer to a biomarker that would be statistically fully validated as having a specific effectiveness in a clinical setting. The biomarker may be a metabolite, a compound, a lipid, a protein, a moiety, a functional group, a composition, a combination of two or more metabolites and/or compounds, a (measurable or measured) quantity thereof, a ratio or other value derived thereof, or in principle any measurement reflecting a chemical and/or biological component that may be found associated with a disease or condition or the risk of having or developing thereof. The biomarkers and any combinations thereof, optionally in combination with further analyses and/or measures, may be used to measure a biological process indicative of the risk for developing a musculoskeletal and/or connective tissue disease, such as rheumatoid arthritis, other arthritis, gout, osteoarthritis, joint disorders, spondylosis and/or soft tissue disorders.

The disease may refer to a category of musculoskeletal and connective tissue diseases or to a specific disease in this category. In the context of this specification, the term "a musculoskeletal and/or connective tissue disease" may be understood as referring to diseases, disorders and/or conditions affecting the musculoskeletal system and/or connective tissue, including, for instance, joints, bones, muscles and associated tissues. It may be a chronic condition, which in the context of this specification may be understood as persistent or otherwise long-lasting in its effects and/or a disease that comes with time. The signs and symptoms of musculoskeletal and/or connective tissue disease may vary from mild to severe or disabling or potentially life-threatening, depending on factors such as age and/or overall health of the subject.

The biomarker associations may be similar for the different musculoskeletal and/or connective tissue diseases. Therefore, the same individual biomarkers and combinations of biomarkers may be extended to also predict the risk for specific musculoskeletal and/or connective tissue diseases. Examples of such specific musculoskeletal and/or connective tissue diseases include rheumatoid arthritis, other arthritis, gout, osteoarthritis, joint disorders, spondylosis and/or soft tissue disorders.

Musculoskeletal and/or connective tissue diseases described in this specification may be classified as follows. "ICD-10" may be understood as referring to the International Statistical Classification of Diseases and Related Health Problems 10th Revision (ICD-10) - WHO Version for 2019. Similar diseases classified or diagnosed by other disease classification systems than ICD-10, such as ICD-9 or ICD-11, may also apply.

The term "Any Musculoskeletal and/or Connective Tissue Disease" may be understood as referring to any musculokeletal and/or connective tissue disease, disorder or condition. Any Musculoskeletal and/or Connective Tissue Disease may be understood as referring to any incident occurrence of ICD-10 diagnoses M00-M99.

Musculoskeletal and/or Connective Tissue Disease Subgroups may, at least in some embodiments, be understood as referring to diseases and/or conditions classified within the ICD-10 subchapter diagnoses for musculoskeletal and/or connective tissue diseases (M05-M14, M15-M19, M20-M25, M45-M49, M50-M54, M65-M67, M70-M79).

Specific musculoskeletal and/or connective tissue diseases may, at least in some embodiments, be understood as referring to diseases and classified within the 3-character ICD-10 diagnoses for musculoskeletal and/or connective tissue diseases (M06, M10, M13, M15, M17, M19, M25, M47, M48, M51, M54, M65, M79).

In an embodiment, the musculoskeletal and/or connective tissue disease is a disease classified within a subgroup of musculoskeletal and/or connective tissue diseases, such as defined by one or more ICD-10 subchapters described in this specification.

In an embodiment, the musculoskeletal and/or connective tissue disease is a specific disease, such as defined by an ICD-10 3-character code diagnosis.

In an embodiment, the musculoskeletal and/or connective tissue diseases is a disease classified among one of the following musculoskeletal and/or connective tissue disease subgroups:

- M05-M14: Inflammatory polyarthropathies
- M15-M19: Osteoarthritis
- M20-M25: Other joint disorders
- M45-M49: Spondylopathies
- M50-M54: Other dorsopathies
- M65-M67: Disorders of synovium and tendon
- M70-M79: Other soft tissue disorders

In an embodiment, the musculoskeletal and/or connective tissue disease is a disease classified within one of the following ICD-10 3-character diagnoses:

- M06: Other rheumatoid arthritis
- M10: Gout
- M13: Other arthritis

- M15: Polyosteoarthritis
- M17: Osteoarthritis of knee
- M19: Other and unspecified osteoarthritis
- M25: Other joint disorder, not elsewhere classified
- M47: Spondylosis
- M48: Other spondylopathies
- M51: Thoracic, thoracolumbar, and lumbosacral intervertebral disc disorders
- M54: Dorsalgia
- M65: Synovitis and tenosynovitis
- M79: Other and unspecified soft tissue disorders, not elsewhere classified

In an embodiment, the musculoskeletal and/or connective tissue disease may comprise or be death from a musculoskeletal and/or connective tissue disease, such as a disease denoted by the ICD-10 codes listed above.

In an embodiment, the musculoskeletal and/or connective tissue disease may comprise or be inflammatory polyarthropathy (M05-M14); osteoarthritis (M15-M19); other joint disorder (M20-M25); spondylopathy (M45-M49); other dorsopathy (M50-M54); disorder of synovium and/or tendon (M65-M67); and/or other soft tissue disorder (M70-M79).

In an embodiment, the musculoskeletal and/or connective tissue disease may comprise or be other rheumatoid arthritis (M06); gout (M10); other arthritis (M13); polyosteoarthritis (M15); osteoarthritis of knee (M17); other and/or unspecified osteoarthritis (M19); other joint disorder, not elsewhere classified (M25); spondylosis (M47); other spondylopathy (M48); thoracic, thoracolumbar, and/or lumbosacral intervertebral disc disorder (M51); dorsalgia (M54); synovitis and/or tenosynovitis (M65); and/or other and unspecified soft tissue disorder, not elsewhere classified (M79) .

In an embodiment, the musculoskeletal and/or connective tissue disease may comprise or rheumatoid arthritis, other arthritis, gout, osteoarthritis, a joint disorder, spondylosis and/or a soft tissue disorder.

The method may further comprise determining whether the subject is at risk of developing a musculoskeletal and/or connective tissue disease using a risk score, hazard ratio, and/or predicted absolute or relative risk calculated on the basis of the quantitative value(s) of the at least one biomarker or of the plurality of the biomarkers.

An increase or a decrease in the risk score, hazard ratio, and/or predicted absolute risk and/or relative risk may be indicative of the subject having an increased risk of developing the musculoskeletal and/or connective tissue disease.

The risk score and/or hazard ratio and/or predicted absolute risk or relative risk may be calculated based on any plurality, combination or subset of biomarkers described in this specification.

The risk score and/or hazard ratio and/or predicted absolute risk or relative risk may be calculated e.g. as shown in the Examples below. For example, the plurality of biomarkers measured using a suitable method, for example with NMR spectroscopy, may be combined using regression algorithms and multivariate analyses and/or using machine learning analysis. Before regression analysis or machine learning, any missing values in the biomarkers may be imputed with the mean value of each biomarker for the dataset. A number of biomarkers, for example five, that may be considered most associated with the onset of the disease or condition may be selected for use in the prediction model. Other modelling approaches may be used to calculate a risk score and/or hazard ratio and/or predicted absolute risk or relative risk based on a combination or subset of individual biomarkers, i.e. a plurality of the biomarkers.

The risk score may be calculated e.g. as a weighted sum of individual biomarkers, i.e. a plurality of the biomarkers. The weighted sum may be e.g. in the form of a multi-biomarker score defined as

$$\sum_i [\beta_i * c_i] + \beta_0;$$

where $i$ is the index of summation over individual biomarkers, $\beta_i$ is the weighted coefficient attributed to biomarker $i$, $c_i$ is the blood concentration (or concentration in the biological sample) of biomarker $i$, and $\beta_0$ is an intercept term.

For example, the risk score can be defined as: $\beta_1$*concentration(glycoprotein acetyls) + $\beta_2$* concentration(mo-nounsaturated fatty acid ratio to total fatty acids) + $\beta_3$* concentration (albumin) + $\beta_0$, where $\beta_1$, $\beta_2$, $\beta_3$ are multipliers for each biomarker according to the association magnitude with risk of a musculoskeletal and/or connective tissue disease and $\beta_0$ is an intercept term. As a skilled person will understand, the biomarkers mentioned in this example may be replaced by any other biomarker(s) described in this specification. In general, the more biomarkers are included in the risk score, the stronger the predictive performance may become. When additional biomarkers are included in the risk score, the $\beta i$ weights may change for all biomarkers according to the optimal combination for the prediction of a musculoskeletal and/or connective tissue disease.

The risk score, hazard ratio, odds ratio, and/or predicted relative risk and/or absolute risk may be calculated on the basis of at least one further measure, for example a characteristic of the subject. Such characteristics may be determined prior to, simultaneously, or after the biological sample is obtained from the subject. As a skilled person will understand, some of the characteristics may be information collected e.g. using a questionnaire or clinical data collected earlier. Some of the characteristics may be determined (or may have been determined) by biochemical or clinical diagnostic measurements and/or medical diagnosis. Such characteristics could include, for example, one or more of age, height, weight, body mass index, race or ethnic group, smoking, and/or family history of musculoskeletal and/or connective tissue diseases.

The method may further comprise administering a treatment to the subject at risk of developing a musculoskeletal and/or connective tissue disease to thereby treat the subject in order to prevent or treat the disease in the subject. The risk prediction for a musculoskeletal and/or connective tissue disease based on one or more of the biomarkers can be used to guide preventative efforts, such as strengthening exercises, stretching, relaxation techniques, non-smoking awareness, healthy diet, and/or clinical screening frequency and/or pharmacological treatment decisions. For example, the information of the future risk for a musculoskeletal and/or connective tissue disease can be used for guiding chiropractic care, therapeutic massage and/or physical or occupational therapy or treatment with, for instance, nonsteroidal anti-inflammatories (NSAIDs), injection with anesthetic or anti-inflammatory medications around the painful sites and/or other anti-inflammatory medications and/or medications to increase the body's level of serotonin and norepinephrine.

In the context of this specification, the term "albumin" may be understood as referring to serum albumin (often referred to as blood albumin). It is an albumin found in vertebrate blood. Albumin is a globular, water-soluble, unglycosylated serum protein of approximate molecular weight of 65,000 Daltons. The measurement of albumin using NMR is described e.g. in publications by Kettunen et al., 2012, Nature Genetics 44, 269-276; Soininen et al., 2015, Circulation: Cardiovascular Genetics 8, 212-206 (DOI: 10.1161/CIRCGENETICS.114.000216). Albumin may also be measured by various other methods, for example by clinical chemistry analyzers. Examples of such methods may include e.g. dye-binding methods such as bromocresol green and bromocresol purple.

In the context of this specification, the term "glycoprotein acetyls", "glycoprotein acetylation", or "GlycA" may refer to the abundance of circulating glycated proteins, and/or to a nuclear magnetic resonance spectroscopy (NMR) signal that represents the abundance of circulating glycated proteins, i.e. N-acetylated glycoproteins. Glycoprotein acetyls may include signals from a plurality of different glycoproteins, including e.g. alpha-1-acid glycoprotein, alpha-1 antitrypsin, haptoglobin, transferrin, and/or alpha-1 antichymotrypsin. In the scientific literature on cardiometabolic biomarkers, the terms "glycoprotein acetyls" or "GlycA" may commonly refer to the NMR signal of circulating glycated proteins (e.g. Ritchie et al, Cell Systems 2015 1(4):293-301; Connelly et al, J Transl Med. 2017;15(1):219). Glycoprotein acetyls and a method for measuring them is described e.g. in Kettunen et al., 2018, Circ Genom Precis Med. 11:e002234 and Soininen et al., 2009, Analyst 134, 1781-1785. There may be benefits of using the NMR signal of glycoprotein acetyls for risk prediction above measurement of the individual proteins contributing to the NMR signal, for instance better analytical accuracy and stability over time, as well as lower costs of the measurement and the possibility to measure the NMR signal simultaneously with many other biomarkers.

In the context of this specification, the term "omega-6 fatty acids" may refer to total omega-6 fatty acids, i.e. the total omega-6 fatty acid amounts and/or concentrations, i.e. the sum of the amounts and/or concentrations of different omega-6 fatty acids. Omega-6 fatty acids are polyunsaturated fatty acids. In omega-6 fatty acids, the last double bond in the fatty acid chain is the sixth bond counting from the methyl end.

In one embodiment, the omega-6 fatty acid may be linoleic acid. Linoleic acid (18:2ω-6) is the most abundant type of omega-6 fatty acids, and may therefore be considered as a good approximation for total omega-6 fatty acids for risk prediction of a musculoskeletal and/or connective tissue disease.

In the context of this specification, the term "monounsaturated fatty acids" (MUFAs) may refer to total monounsaturated fatty acids, i.e. the total MUFA amounts and/or concentrations. Monounsaturated fatty acids may, alternatively, refer to oleic acid, which is the most abundant monounsaturated fatty acid in human serum. Monounsaturated fatty acids have one double bond in their fatty acid chain. The monounsaturated fatty acids may include omega-9 and omega-7 fatty acids. Oleic acid (18:1ω-9), palmitoleic acid (16:1ω-7) and cis-vaccenic acid (18:1ω-7) are examples of common monounsaturated fatty acids in human serum.

In one embodiment, the monounsaturated fatty acid may be oleic acid. Oleic acid is the most abundant monounsaturated fatty acid, and may therefore be considered as a good approximation for total monounsaturated fatty acids for risk prediction of a musculoskeletal and/or connective tissue disease.

In the context of this specification, the term "saturated fatty acids" (SFAs) may refer to total saturated fatty acids. Saturated fatty acids may be or comprise fatty acids which have no double bonds in their structure. Palmitic acid (16:0) and stearic acid (18:0) are examples of abundant SFAs in human serum.

For all fatty acid measures, including omega-6, docosahexaenoic acid, linoleic acid, monounsaturated fatty acids and/or saturated fatty acids, the fatty acid measures may include blood (or serum/plasma) free fatty acids, bound fatty acids and esterified fatty acids. Esterified fatty acids may, for example, be esterified to glycerol as in triglycerides, diglycerides, monoglycerides, or phosphoglycerides, or to cholesterol as in cholesterol esters.

In the context of this specification, the term "fatty acid degree of unsaturation" or "unsaturation" may be understood as referring to the number of double bonds in total fatty acids, for example the average number of double bonds in total fatty acids.

In the context of this specification, the term "" refers to high-density lipoprotein.

In the context of this specification, the term "LDL" refers to low-density lipoprotein.

In the context of this specification, the term "VLDL" refers to very-low-density lipoprotein.

In the context of this specification, the phrase "low-density lipoprotein (LDL) triglycerides", "high-density lipoprotein (HDL) triglycerides", "triglycerides in HDL (high-density lipoprotein)", or "triglycerides in LDL (low-density lipoprotein)", may be understood as referring to total triglyceride concentration in said lipoprotein class or subfraction.

In the context of this specification, the phrase "high-density lipoprotein (HDL) particle size", "low-density lipoprotein (LDL) particle size", or "very-low-density lipoprotein (VLDL) particle size", may be understood as referring to the average diameter for the particles in said lipoprotein class or subfraction.

In the context of this specification, the term "acetate" may refer to the acetate molecule and/or acetic acid, for example in blood, plasma or serum or related biofluids.

In the context of this specification, the term "citrate" may refer to the citrate molecule and/or citric acid, for example in blood, plasma or serum or related biofluids.

In the context of this specification, the term "glutamine" may refer to the glutamine amino acid, for example in blood, plasma or serum or related biofluids.

In the context of this specification, the term "glycine" may refer to the glycine amino acid, for example in blood, plasma or serum or related biofluids.

In the context of this specification, the term "histidine" may refer to the histidine amino acid, for example in blood, plasma or serum or related biofluids.

In the context of this specification, the term "isoleucine" may refer to the isoleucine amino acid, for example in blood, plasma or serum or related biofluids.

In the context of this specification, the term "leucine" may refer to the leucine amino acid, for example in blood, plasma or serum or related biofluids.

In the context of this specification, the term "phenylalanine" may refer to the phenylalanine amino acid, for example in blood, plasma or serum or related biofluids.

In the context of this specification, the term "valine" may refer to the valine amino acid, for example in blood, plasma or serum or related biofluids.

In the context of this specification, the term "quantitative value" may refer to any quantitative value characterizing the amount and/or concentration of a biomarker. For example, it may be the amount or concentration of the biomarker in the biological sample, or it may be a signal derived from nuclear magnetic resonance spectroscopy (NMR) or other method suitable for detecting the biomarker in a quantitative manner. Such a signal may be indicative of or may correlate with the amount or concentration of the biomarker. It may also be a quantitative value calculated from one or more signals derived from NMR measurements or from other measurements. Quantitative values may, additionally or alternatively, be measured using a variety of techniques. Such methods may include mass spectrometry (MS), gas chromatography combined with MS, high performance liquid chromatography alone or combined with MS, immunoturbidimetric measurements, ultracentrifugation, ion mobility, enzymatic analyses, colorimetric or fluorometric analyses, immunoblot analysis, immunohistochemical methods (e.g. *in situ* methods based on antibody detection of metabolites), and immunoassays (e.g. ELISA). Examples of various methods are set out below. The method used to determine the quantitative value(s) in the subject may be the same method that is used to determine the quantitative value(s) in a control subject/control subjects or in a control sample/control samples.

The quantitative value, or the initial quantitative value, of the at least one biomarker, or the plurality of the biomarkers, may be measured using nuclear magnetic resonance (NMR) spectroscopy, for example $^1$H-NMR. The at least one additional biomarker, or the plurality of the additional biomarkers, may also be measured using NMR. NMR may provide a particularly efficient and fast way to measure biomarkers, including a large number of biomarkers simultaneously, and can provide quantitative values for them. NMR also typically requires very little sample pre-treatment or preparation. The biomarkers measured with NMR can effectively be measured for large amounts of samples using an assay for blood (serum or plasma) NMR metabolomics previously published by Soininen et al., 2015, Circulation: Cardiovascular Genetics 8, 212-206 (DOI: 10.1161/CIRCGENETICS.114.000216) ; Soininen et al., 2009, Analyst 134, 1781-1785; and Würtz et al., 2017, American Journal of Epidemiology 186 (9), 1084-1096 (DOI: 10.1093/aje/kwx016). This provides data on 250 biomarkers per sample as described in detail in the above scientific papers.

In an embodiment, the (initial) quantitative value of the at least one biomarker is/are measured using nuclear magnetic resonance spectroscopy.

However, quantitative values for various biomarkers described in this specification may also be performed by techniques other than NMR. For example, mass spectrometry (MS), enzymatic methods, antibody-based detection methods, or other biochemical or chemical methods may be contemplated, depending on the biomarker.

For example, glycoprotein acetyls can be measured or approximated by immunoturbidimetric measurements of alpha-1-acid glycoprotein, haptoglobin, alpha-1-antitrypsin, and transferrin (e.g. as described in Ritchie et al., 2015, Cell Syst. 28; 1(4) :293-301) .

E.g. monounsaturated fatty acids, saturated fatty acids, and omega-6 fatty acids can be quantified (i.e. their quantitative values may be determined) by serum total fatty acid composition using gas chromatography (for example, as described in Jula et al., 2005, Arterioscler Thromb Vasc Biol 25, 2152-2159).

In the context of this specification, the term "sample" or "biological sample" may refer to any biological sample obtained from a subject or a group or population of subjects. The sample may be fresh, frozen, or dry.

The biological sample may comprise or be, for example, a blood sample, a plasma sample, a serum sample, or a sample derived therefrom. The biological sample may be, for example, a fasting blood sample, a fasting plasma sample, a fasting serum sample, or a fraction obtainable therefrom. However, the biological sample does not necessarily have to be a fasting sample. The blood sample may be a venous blood sample.

The blood sample may be a dry blood sample. The dry blood sample may be a dried whole blood sample, a dried plasma sample, a dried serum sample, or a dried sample derived therefrom.

The method may comprise obtaining the biological sample from the subject prior to determining the quantitative value of the at least one biomarker. Taking a blood sample or a tissue sample of a subject or patient is a part of normal clinical practice. The collected blood or tissue sample can be prepared and serum or plasma can be separated using techniques well known to a skilled person. Methods for separating one or more fractions from biological samples, such as blood samples or tissue samples, are also available to a skilled person. The term "fraction" may, in the context of this specification, also refer to a portion or a component of the biological sample separated according to one or more physical properties, for instance solubility, hydrophilicity or hydrophobicity, density, or molecular size.

In the context of this specification, the term "control sample" may refer to a sample obtained from a subject and known not to suffer from the disease or condition or not being at risk of having or developing the disease or condition. The control sample may be matched. In an embodiment, the control sample may be a biological sample from a healthy individual or a generalized population of healthy individuals. The term "control value" may be understood as a value obtainable from the control sample and/or a quantitative value derivable therefrom. For example, it may be possible to calculate a threshold value from control samples and/or control values, above or below which the risk of developing the disease or condition is elevated. In other words, a value higher or lower (depending on the biomarker, risk score, hazard ratio, and/or predicted absolute risk or relative risk) than the threshold value may be indicative of the subject having an increased risk of developing the disease or condition.

An increase or a decrease in the quantitative value(s) of the at least one biomarker, or the plurality of the biomarkers, when compared to the control sample or to the control value, may be indicative of the subject having an increased risk of having or developing the disease or condition. Whether an increase or a decrease is indicative of the subject having an increased risk of developing the disease or condition, may depend on the biomarker.

A 1.2-fold, 1.5-fold, or for example 2-fold, or 3-fold, increase or a decrease in the quantitative value (s) of the at least one biomarker (or in an individual biomarker of the plurality of the biomarkers) when compared to the control sample or to the control value, may be indicative of the subject having an increased risk of developing the disease or condition.

In an embodiment, a decrease in the quantitative value of albumin, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, an increase in the quantitative value of glycoprotein acetyls, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of docosahexaenoic acid ratio to total fatty acids, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of linoleic acid ratio to total fatty acids, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, an increase in the quantitative value of the ratio of monounsaturated fatty acid and/or of oleic acid to total fatty acids, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of the ratio of omega-6 fatty acids and/or of linoleic acid to total fatty acids, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, an increase in the quantitative value of the ratio of saturated fatty acids to total fatty acids, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of fatty acid degree of unsaturation, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of docosahexaenoic acid, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of linoleic acid, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, an increase in the quantitative value of monounsaturated fatty acids, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of omega-6 fatty acids, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, an increase in the quantitative value of saturated fatty acids, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, an increase in the quantitative value of triglycerides in high-density lipoprotein (HDL), when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, an increase in the quantitative value of triglycerides in low-density lipoprotein (LDL), when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of high-density lipoprotein (HDL) particle size, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of low-density lipoprotein (LDL) particle size, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, an increase in the quantitative value of very-low-density lipoprotein (VLDL) particle size, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of acetate, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of citrate, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of glutamine, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of glycine, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a decrease in the quantitative value of histidine, when compared to the control sample or to the control value, may be indicative of the subject having an increased risk of a musculoskeletal and/or connective tissue disease, such as rheumatoid arthritis, other arthritis, gout, osteoarthritis, a joint disorder, spondylosis and/or a soft tissue disorder.

In an embodiment, an increase in the quantitative value of isoleucine, when compared to the control sample or to the control value, may be indicative of the subject having an increased risk of a musculoskeletal and/or connective tissue disease, such as rheumatoid arthritis, other arthritis, gout, osteoarthritis, a joint disorder, spondylosis and/or a soft tissue disorder.

In an embodiment, an increase in the quantitative value of leucine, when compared to the control sample or to the control value, may be indicative of the subject having an increased risk of a musculoskeletal and/or connective tissue disease, such as rheumatoid arthritis, other arthritis, gout, osteoarthritis, a joint disorder, spondylosis and/or a soft tissue disorder.

In an embodiment, an increase in the quantitative value of phenylalanine, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, an increase in the quantitative value of valine, when compared to the control sample or to the control value, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a risk score defined as $\beta_0 + \beta_1$* concentration (glycoprotein acetyls) + $\beta_2$* concentration (albumin), where $\beta_0$ is an intercept term, $\beta_1$ is the weighted coefficient attributed to the concentration of glycoprotein acetyls, and $\beta_2$ is the weighted coefficient attributed to the concentration of albumin, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder.

In an embodiment, a risk score defined as $\beta_0 + \beta_1$* concentration (glycoprotein acetyls) + $\beta_2$* concentration (fatty acid measure), where $\beta_0$ is an intercept term, $\beta_1$ is the weighted coefficient attributed to the concentration of glycoprotein acetyls, $\beta_2$ is the weighted coefficient attributed to the fatty acid measure, may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder. The fatty acid measure may be one or more of the following fatty acids or their ratio to total fatty acids: docosahexaenoic acid, linoleic acid, omega-6 fatty acids, monounsaturated fatty acids, saturated fatty acids and/or fatty acid degree of unsaturation.

In an embodiment, a risk score defined as $\beta_0 + \beta_1$* concentration (glycoprotein acetyls) + $\beta_2$* concentration (albumin) + $\beta_3$* concentration (fatty acid measure), where $\beta_0$ is an intercept term, $\beta_1$ is the weighted coefficient attributed to the concentration of glycoprotein acetyls, $\beta_2$ is the weighted coefficient attributed to the concentration of albumin, and $\beta_3$ is the weighted coefficient attributed to the concentration of the fatty acid measure may be indicative of the subject having an increased risk of developing a musculoskeletal and/or connective tissue disease, such as rheumatoid arthritis, other arthritis, gout, osteoarthritis, a joint disorder, spondylosis and/or a soft tissue disorder. The fatty acid measure may be one or more of the following fatty acids or their ratio to total fatty acids: docosahexaenoic acid, linoleic acid, omega-6 fatty acids, monounsaturated fatty acids, saturated fatty acids and/or fatty acid degree of unsaturation.

The term "combination" may, at least in some embodiments, be understood such that the method comprises using a risk score, hazard ratio, odds ratio, and/or predicted absolute risk or relative risk calculated on the basis of the quantitative value(s) of the biomarkers. For example, if quantitative values of both glycoprotein acetyls and albumin are determined, the quantitative values of both biomarkers may be compared to the control sample or the control value separately, or a risk score, hazard ratio, odds ratio, and/or predicted absolute risk or relative risk calculated on the basis of the quantitative value(s) of both the biomarkers, and the risk score, hazard ratio, odds ratio, and/or predicted absolute risk or relative risk may be compared to the control sample or the control value.

In an embodiment, the method may comprise determining in the biological sample obtained from the subject a quantitative value of the following biomarkers:

- glycoprotein acetyls;

- albumin; and

comparing the quantitative value(s) of the biomarkers and/or a combination thereof to a control sample or to a control value(s);
wherein an increase or a decrease in the quantitative value(s) of the biomarkers and/or the combination thereof, when compared to the control sample or to the control value, is/are indicative of the subject having an increased risk of developing the musculoskeletal and/or connective tissue disease. An increase in the quantitative value of glycoprotein acetyls and a decrease in the quantitative value of albumin, when compared to the control sample or to the control value, may be indicative of the subject having an increased risk of developing the musculoskeletal and/or connective tissue disease.

In an embodiment, the method may comprise determining in the biological sample obtained from the subject a quantitative value of the following biomarkers:

- glycoprotein acetyls,
- at least one fatty acid measure(s) of the following fatty acids or their ratio to total fatty acids: docosahexaenoic acid, linoleic acid, omega-6 fatty acids, monounsaturated fatty acids, saturated fatty acids and/or fatty acid degree of unsaturation; and

comparing the quantitative value(s) of the biomarkers and/or a combination thereof to a control sample or to a control value(s);
wherein an increase or a decrease in the quantitative value(s) of the biomarkers and/or the combination thereof, when compared to the control sample or to the control value, is/are indicative of the subject having an increased risk of developing the musculoskeletal and/or connective tissue disease. An increase in the quantitative value of glycoprotein acetyls and a decrease in the quantitative value of docosahexaenoic and/or linoleic acid and/or omega-6 fatty acids and/or fatty acid degree of unsaturation and/or their ratio to total fatty acids, and/or an increase in the quantitative value of monounsaturated fatty acids and/or saturated fatty acids and/or their ratio to total fatty acids, when compared to the control sample or to the control value, may be indicative of the subject having an increased risk of developing the musculoskeletal and/or connective tissue disease.

In an embodiment, the method may comprise determining in the biological sample obtained from the subject a quantitative value of the following biomarkers:

- albumin,
- at least one fatty acid measure(s) of the following fatty acids or their ratio to total fatty acids: docosahexaenoic acid, linoleic acid, omega-6 fatty acids, monounsaturated fatty acids, saturated fatty acids and/or fatty acid degree of unsaturation; and

comparing the quantitative value(s) of the biomarkers and/or a combination thereof to a control sample or to a control value(s);
wherein an increase or a decrease in the quantitative value(s) of the biomarkers and/or the combination thereof, when compared to the control sample or to the control value, is/are indicative of the subject having an increased risk of developing the musculoskeletal and/or connective tissue disease. A decrease in the quantitative value of albumin and a decrease in the quantitative value of docosahexaenoic and/or linoleic acid and/or omega-6 fatty acids and/or fatty acid degree of unsaturation and/or their ratio to total fatty acids, and/or an increase in the quantitative value of monounsaturated fatty acids and/or saturated fatty acids and/or their ratio to total fatty acids, when compared to the control sample or to the control value, may be indicative of the subject having an increased risk of developing the musculoskeletal and/or connective tissue disease.

In an embodiment, the method may comprise determining in the biological sample obtained from the subject a quantitative value of the following biomarkers:

- glycoprotein acetyls,
- albumin,
- at least one fatty acid measure(s) of the following fatty acids or their ratio to total fatty acids: docosahexaenoic acid, linoleic acid, omega-6 fatty acids, monounsaturated fatty acids, saturated fatty acids and/or fatty acid degree of unsaturation; and

comparing the quantitative value(s) of the biomarkers and/or a combination thereof to a control sample or to a control value(s);

wherein an increase or a decrease in the quantitative value(s) of the biomarkers and/or the combination thereof, when compared to the control sample or to the control value, is/are indicative of the subject having an increased risk of developing the musculoskeletal and/or connective tissue disease. An increase in the quantitative value of glycoprotein acetyls, a decrease in the quantitative value of albumin and a decrease in the quantitative value of docosahexaenoic and/or linoleic acid and/or omega-6 fatty acids and/or fatty acid degree of unsaturation and/or their ratio to total fatty acids, and/or an increase in the quantitative value of monounsaturated fatty acids and/or saturated fatty acids and/or their ratio to total fatty acids, when compared to the control sample or to the control value, may be indicative of the subject having an increased risk of developing the musculoskeletal and/or connective tissue disease.

EXAMPLES

Reference will now be made in detail to various embodiments, an example of which is illustrated in the accompanying drawings. The description below discloses some embodiments in such a detail that a person skilled in the art is able to utilize the embodiments based on the disclosure. Not all steps or features of the embodiments are discussed in detail, as many of the steps or features will be obvious for the person skilled in the art based on this specification.

**Abbreviations used in the Figures:**

DHA %: Ratio of docosahexaenoic acid to total fatty acids
LA%: Ratio of linoleic acid to total fatty acids
MUFA %: Ratio of monounsaturated fatty acids to total fatty acids
Omega-6 %: Ratio of omega-6 fatty acids to total fatty acids
SFA %: Ratio of saturated fatty acids to total fatty acids
DHA: Docosahexaenoic acid
LA: Linoleic acid
MUFA: Monounsaturated fatty acids
Omega-6: Omega-6 fatty acids
SFA: Saturated fatty acids
Unsaturation: Fatty acid degree of unsaturation
HDL: High-density lipoprotein
LDL: Low-density lipoprotein
VLDL: Very-low-density lipoprotein
HDL-TG: Triglycerides in high-density lipoprotein (HDL)
LDL-TG: Triglycerides in low-density lipoprotein (LDL)
CI: confidence interval
SD: standard deviation
BMI: Body mass index

**EXAMPLE 1**

Biomarker measures quantified by nuclear magnetic resonance (NMR) were investigated as to whether they could be predictive of a musculoskeletal and/or connective tissue disease, such as rheumatoid arthritis, other arthritis, gout, osteoarthritis, joint disorders, spondylosis and/or soft tissue disorders. All analyses were conducted based on the UK Biobank, with approximately 115 000 study participants with blood biomarker data from NMR spectroscopy available.

Study population

Details of the design of the UK Biobank have been reported by Sudlow et al 2015, PLoS Med. 2015;12(3):e1001779. Briefly, UK Biobank recruited 502 639 participants aged 37-73 years in 22 assessment centres across the UK. All participants provided written informed consent and ethical approval was obtained from the North West Multi-Center Research Ethics Committee. Blood samples were drawn at baseline between 2007 and 2010. No selection criteria were applied to the sampling.

Biomarker profiling

From the entire UK Biobank population, a random subset of baseline plasma samples from 118 466 individuals were measured using the Nightingale NMR biomarker platform (Nightingale Health Ltd, Finland). This blood analysis method provides simultaneous quantification of many blood biomarkers, including lipoprotein lipids, circulating fatty acids, and various low-molecular weight metabolites including amino acids, ketone bodies and gluconeogenesis-related metabolites in molar concentration units. Technical details and epidemiological applications have been reviewed (Soininen et al 2015, Circ Cardiovasc Genet; 2015;8:192-206; Würtz et al 2017, Am J Epidemiol 2017;186:1084-1096). Values outside four interquartile ranges from median were considered as outliers and excluded.

**Epidemiological analyses of biomarker relations with the risk of a musculoskeletal and/or connective tissue disease**

The blood biomarker associations with the risk for a musculoskeletal and/or connective tissue disease were conducted based on UK Biobank data. Analyses focused on the relation of the biomarkers to the occurrence of a musculoskeletal and/or connective tissue disease after the blood samples were collected, to determine if the individual biomarkers associate with the risk for future development of a musculoskeletal and/or connective tissue disease. Examples using multi-biomarker scores, in the form of weighted sums of biomarkers, were also explored to see if they could be predictive even more strongly than each individual biomarker.

Information on the disease events occurring after the blood samplings for all study participants were recorded from UK Hospital Episode Statistics data and death registries. All analyses are based on first occurrence of diagnosis, so that individuals with recorded diagnosis of the given disease prior to blood sampling were omitted from the statistical analyses. A composite endpoint of Any Musculoskeletal and/or Connective Tissue Disease was defined based on any incident occurrence of ICD-10 diagnoses M00-M99. More refined subtypes of the musculoskeletal and/or connective tissue diseases were defined according to the ICD-10 diagnoses listed in Table 1.

The registry-based follow-up was from blood sampling in 2007-2010 through to 2020 (approximately 1 100 000 person-years). Specific diseases which had <1000 disease events recorded during follow-up were left out of scope.

For biomarker association testing, Cox proportional-hazard regression models adjusted for age, sex, and UK Biobank assessment centre were used. Results were plotted in magnitudes per standard deviation of each biomarker measure to allow direct comparison of association magnitudes.

**Summary of results**

Baseline characteristics of the study population for biomarker analyses vs future risk of a musculoskeletal and/or connective tissue disease are shown in Table 1. The number of incident disease events occurring after the blood sampling is listed for all the conditions analysed.

## Table 1: Clinical characteristics of study participants and the number of incident disease events analysed.

| | |
|---|---|
| **Total number of individuals with blood samples analysed** | 118 456 |
| **Study setting** | Population sample of study volunteers from the UK |
| Percentage of women | 54.1% |

| | |
|---|---|
| Age range (years) | 39 - 71 |
| Median age (years) | 58 |
| Median BMI (kg/m2) | 26.8 |
| Follow-up time for disease events after blood sampling | 10-14 years |
| **Diseases with similar biomarker relations** | Number of individuals who developed the specified disease after the blood sampling |
| **Any Musculoskeletal and/or Connective Tissue Disease: any occurrence of M00-M99 ICD-10 codes** | 22 986 |
| **Musculoskeletal and/or Connective Tissue Disease Subgroups (defined by ICD-10 subchapters)** | |
| M05-M14: Inflammatory polyarthropathies | 84 06 |
| M15-M19: Osteoarthritis | 14 945 |
| M20-M25: Other joint disorders | 8 183 |
| M45-M49: Spondylopathies | 4 922 |
| M50-M54: Other dorsopathies | 5 945 |
| M65-M67: Disorders of synovium and tendon | 2 172 |
| M70-M79: Other soft tissue disorders | 7 478 |
| **Specific Musculoskeletal and/or Connective Tissue Diseases (defined by 3-character ICD-10 codes)** | |
| M06: Other rheumatoid arthritis | 1 457 |
| M10: Gout | 1 748 |
| M13: Other arthritis | 5 802 |
| M15: Polyosteoarthritis | 2 141 |
| M17: Osteoarthritis of knee | 6 276 |
| M19: Other and unspecified osteoarthritis | 7 625 |
| M25: Other joint disorder, not elsewhere classified | 3 911 |

| | |
|---|---|
| M47: Spondylosis | 3 295 |
| M48: Other spondylopathies | 1 928 |
| M51: Thoracic, thoracolumbar, and lumbosacral intervertebral disc disorders | 2 260 |
| M54: Dorsalgia | 4 505 |
| M65: Synovitis and tenosynovitis | 1 386 |
| M79: Other and unspecified soft tissue disorders, not elsewhere classified | 3 858 |

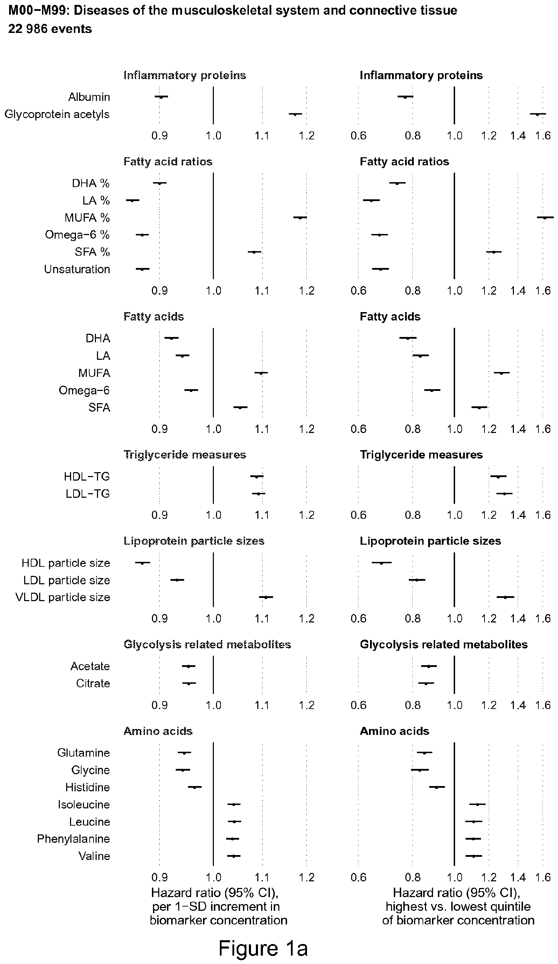

**Figure 1a** shows the hazard ratios for the 27 blood biomarkers with the future risk of Any Musculoskeletal and/or Connective Tissue Disease (ICD-10 codes M00-M99). The left-hand side of the figure shows the hazard ratios when the biomarkers are analysed in absolute concentrations, scaled to standard deviations of the study population. The right-hand side shows the corresponding hazard ratios when individuals in the highest quintile of the biomarker concentration are compared to those in the lowest quintile. The results are based on statistical analyses of over 115 000 individuals from the UK Biobank, out of whom 22 986 developed a musculoskeletal and/or connective tissue disease (defined as diagnoses M00-M99 in the hospital registries, or in the death records) during approximately 10 years of follow-up. The analyses were adjusted for age, sex, and UK Biobank assessment centre in Cox proportional-hazard regression models. P-values were P<0.0001 (corresponding to multiple testing correction) for all associations. These results demonstrate that the 27 individual biomarkers are predictive of the risk for a musculokeletal and/or connective tissue disease in general population settings.

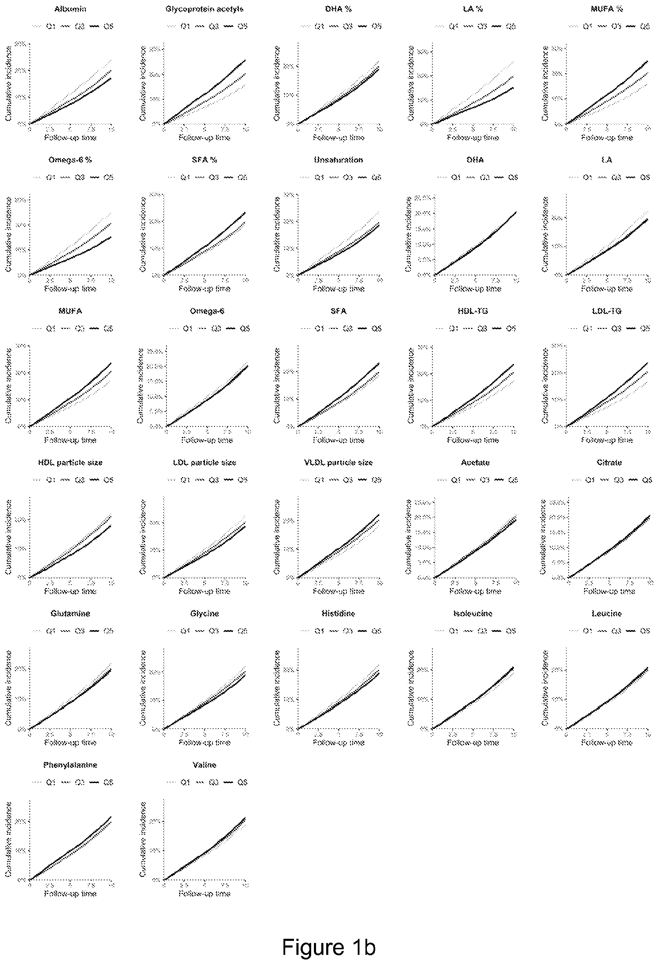

**Figure 1b** shows the Kaplan-Meier plots of the cumulative risk for a musculoskeletal and/or connective tissue disease for each of the 27 blood biomarkers according to the lowest, middle, and highest quintiles of biomarker concentrations. The results are based on statistical analyses of over 115 000 individuals from the UK Biobank, out of whom 22 986 developed a musculoskeletal and/or connective tissue disease. These results further demonstrate that the 27 individual biomarkers are predictive of the risk for a musculoskeletal and/or connective tissue disease in general population settings.

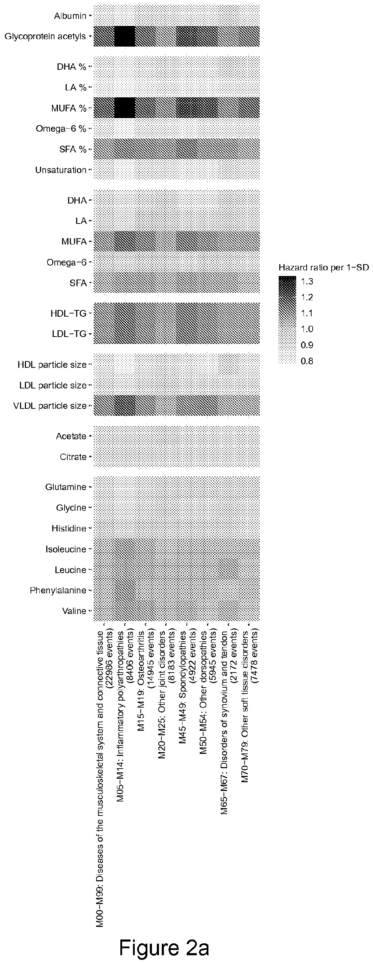

**Figure 2a** shows the hazard ratios for the 27 blood biomarkers for the future onset of 7 subgroups of musculoskeletal and/or connective tissue diseases, defined by ICD-10 subchapters. The results illustrate that the pattern of biomarker associations is highly consistent for the 7 different subtypes of musculoskeletal and/or connective tissue diseases.

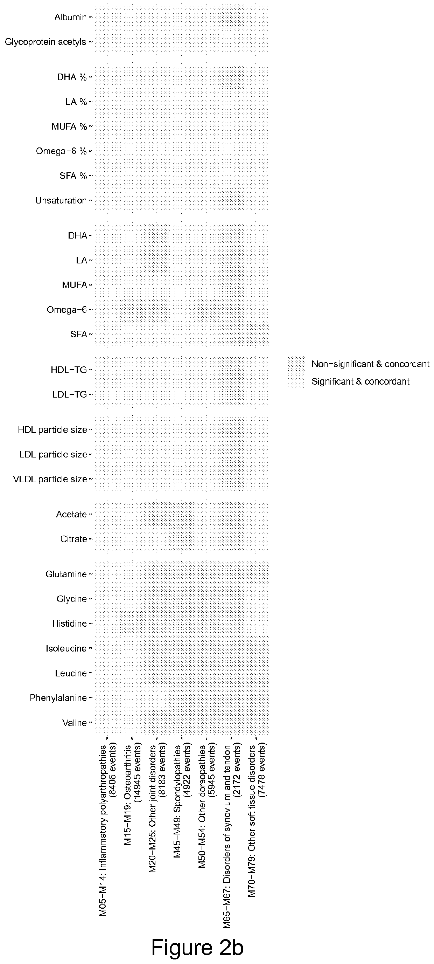

**Figure 2b** shows the consistency of the biomarker associations with the 7 musculoskeletal and/or connective tissue disease subgroups (defined by ICD-10 subchapters) compared to the "Any Musculoskeletal and/or Connective Tissue Disease" definition. The biomarker associations were all in the same direction of association as for "Any Musculoskeletal and/or Connective Tissue Disease" or not statistically significant in the discordant direction. Any biomarker combination that strongly predicts "Any Musculoskeletal and/or Connective Tissue Disease" will therefore also be predictive of all the listed musculoskeltal and/or connective tissue disease subgroups.

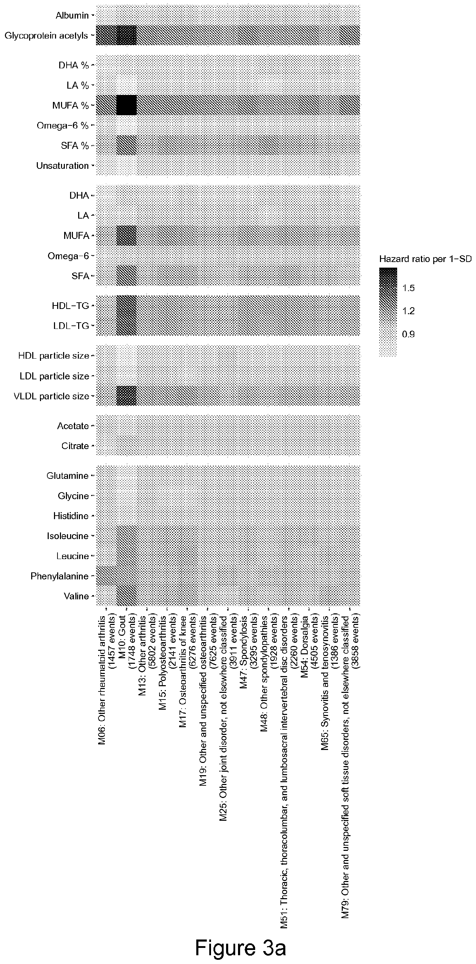

**Figure 3a** shows the hazard ratios for the 27 blood biomarkers for future onset of 13 specific musculoskeletal and/or connective tissue diseases, defined by 3-character ICD-10 diagnosis codes. The results illustrate that the pattern of biomarker associations is highly consistent for all the 13 specific disorders.

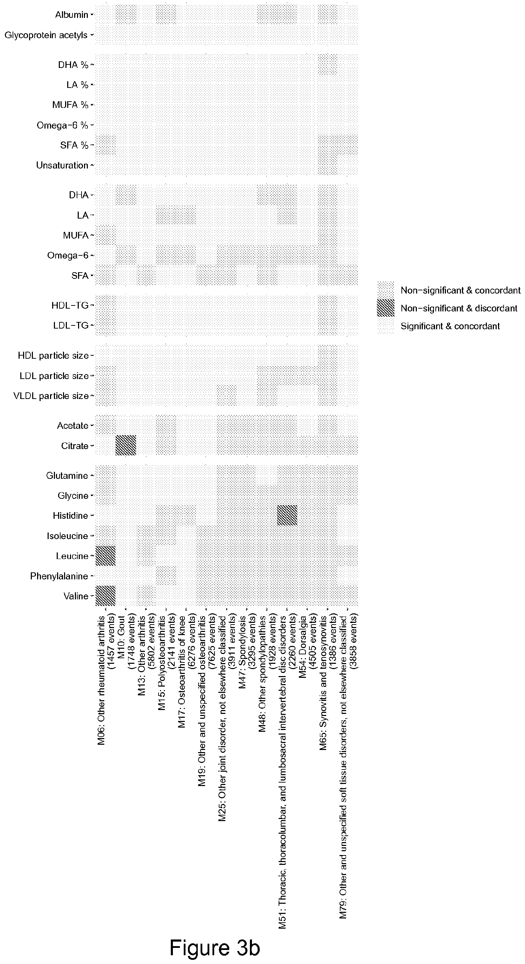

**Figure 3b** shows the consistency of the biomarker associations with the 13 specific musculoskeletal and/or connective tissue diseases (defined by 3-character ICD-10 diagnosis codes) compared to the "Any Musculoskeletal and/or Connective Tissue Disease" definition. Generally, the biomarker associations are all in the same direction of association as for "Any Musculoskeletal and/or Connective Tissue Disease" or not statistically significant in the discordant direction. Any biomarker combination that strongly predicts "Any Musculoskeletal and/or Connective Tissue Disease" will therefore also be predictive of all the listed specific musculoskeletal and/or connective tissue diseases.

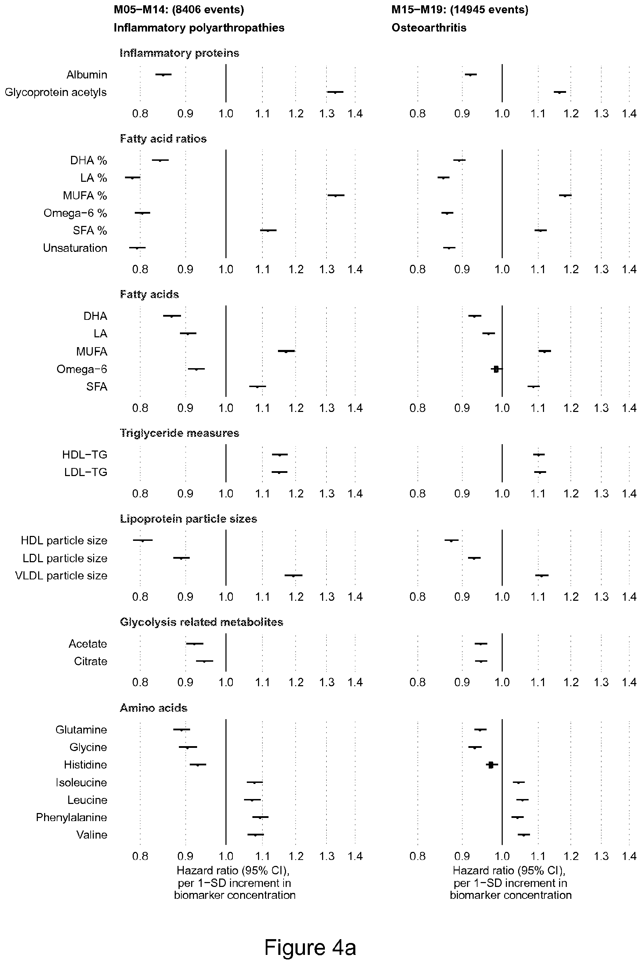

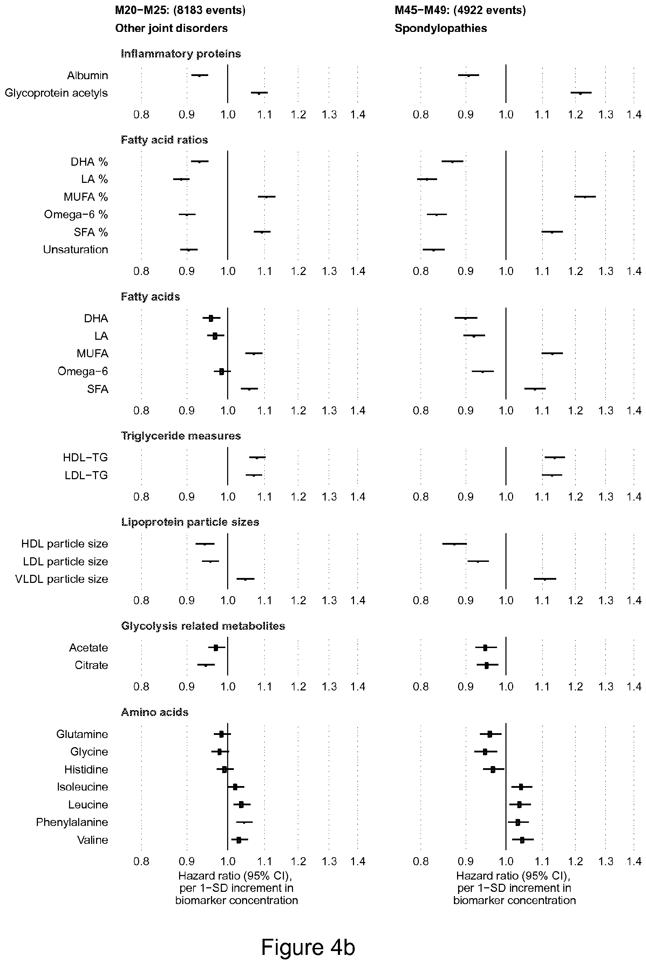

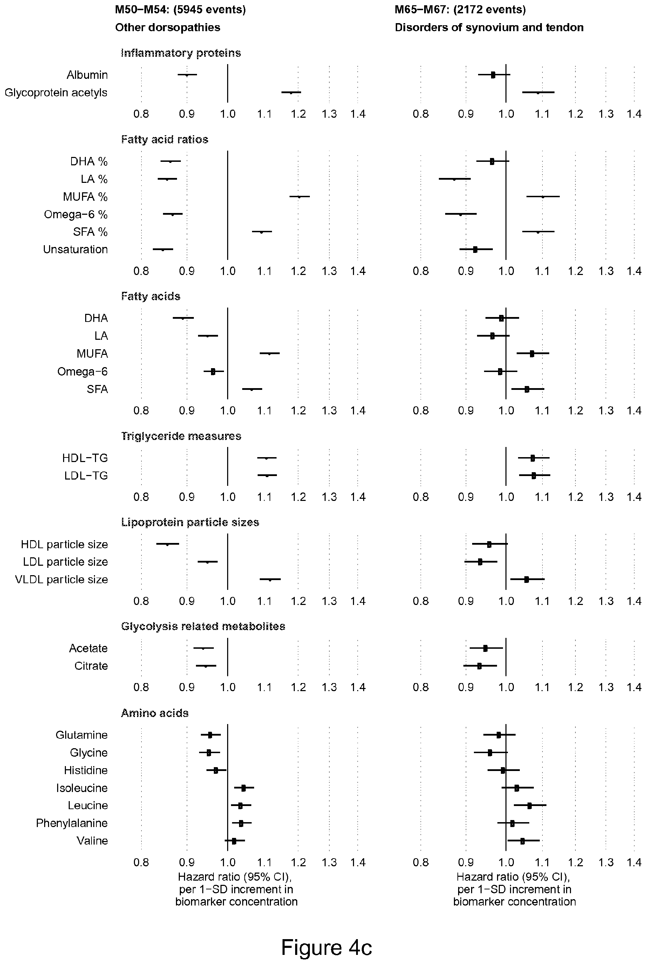

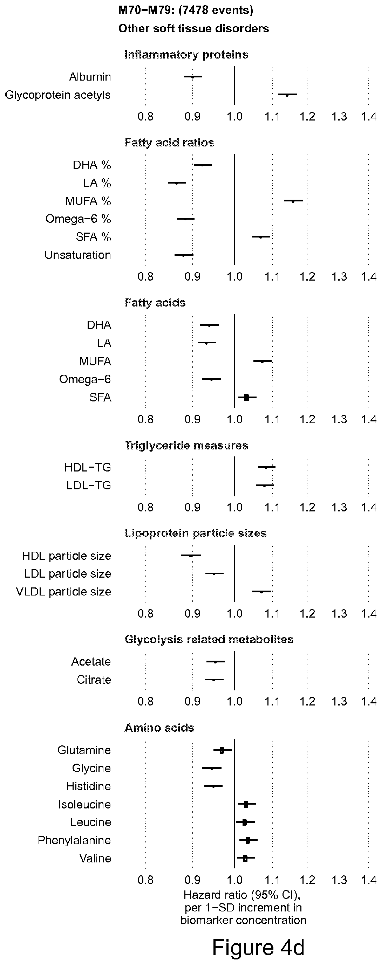

**Figures 4a-d** show the hazard ratios for the 27 blood biomarkers with future onset of each of the 7 musculoskeletal and/or connective tissue disease subgroups (defined by ICD-10 subchapters) studied here. The hazard ratios are shown in absolute concentrations, scaled to the standard deviation of each biomarker. The results are based on statistical analyses of over 115 000 individuals from the UK Biobank; the number of individuals who developed the disease during approximately 10 years of follow-up is indicated on the top of each plot. Filled circles denote that the P-value for association was P<0.0001 (corresponding to multiple testing correction), and open circles denote that the P-value for association was P≥0.0001. The analyses were adjusted for age, sex, and UK Biobank assessment centre using Cox proportional-hazard regression models.

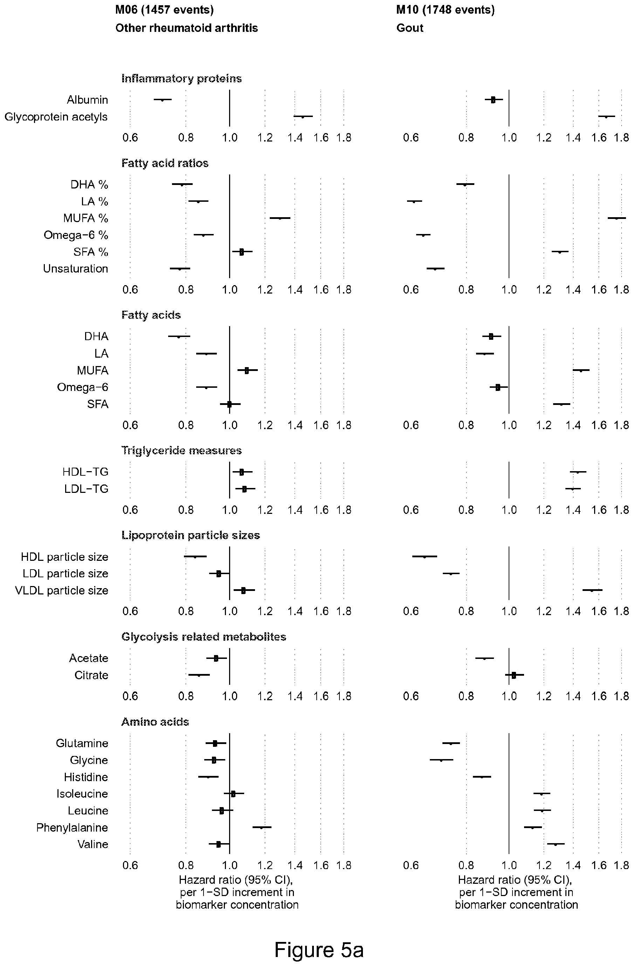

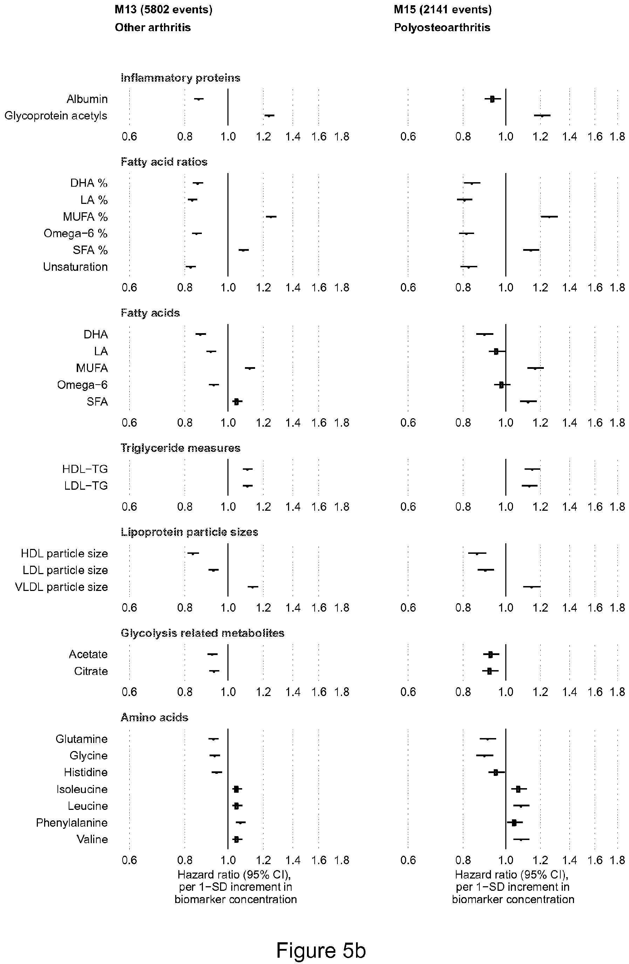

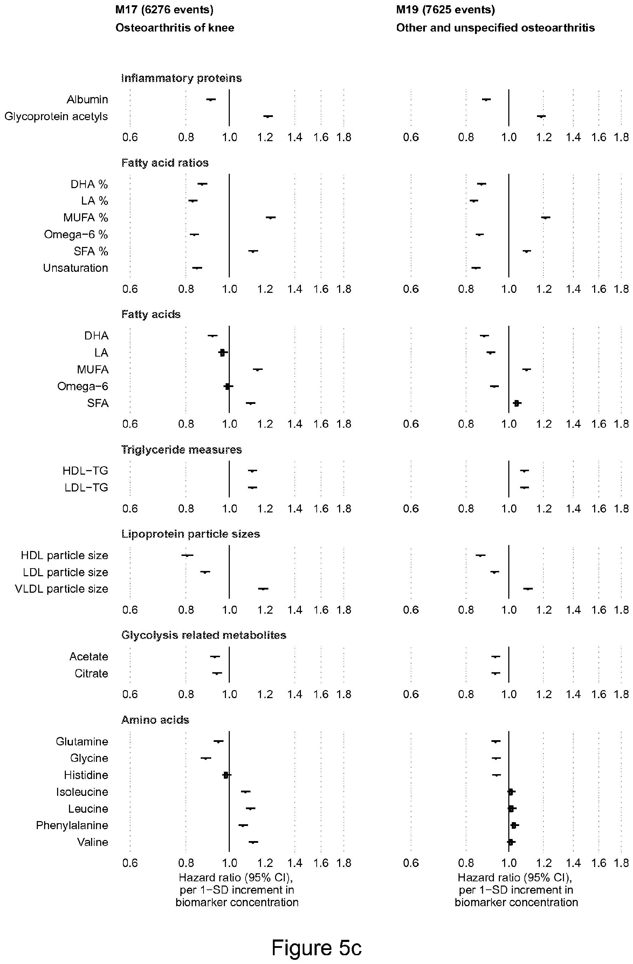

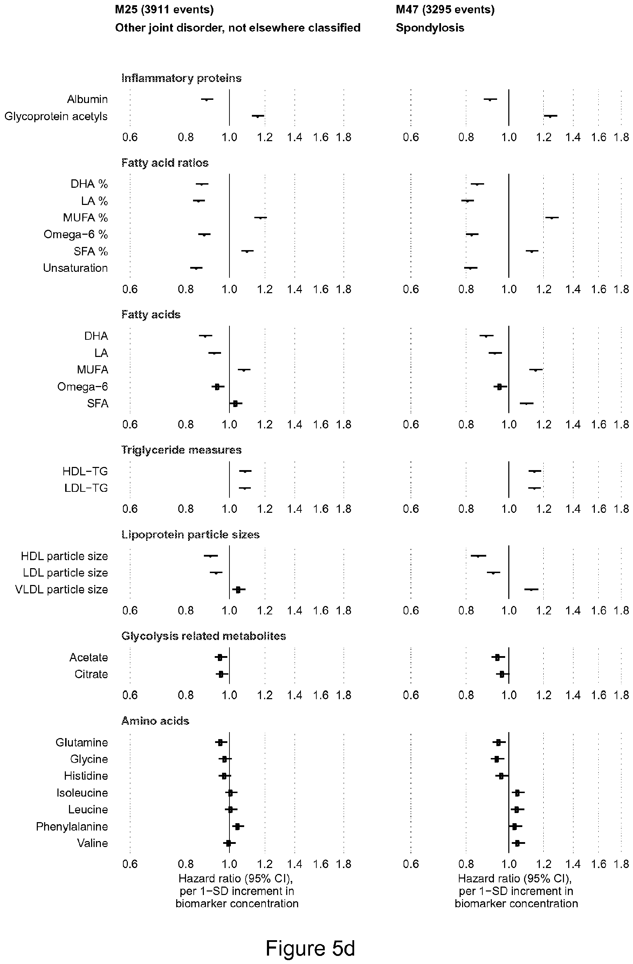

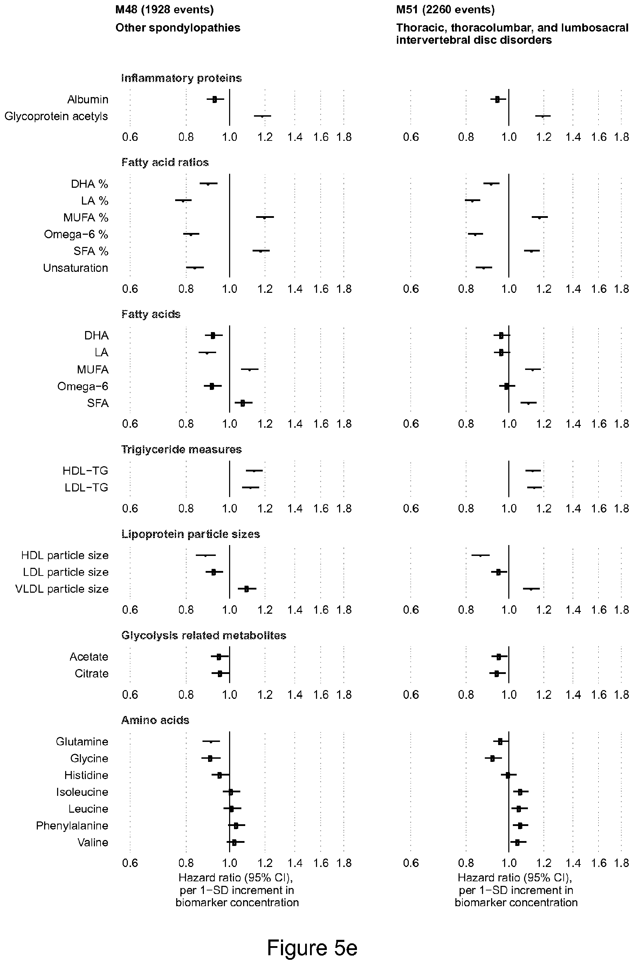

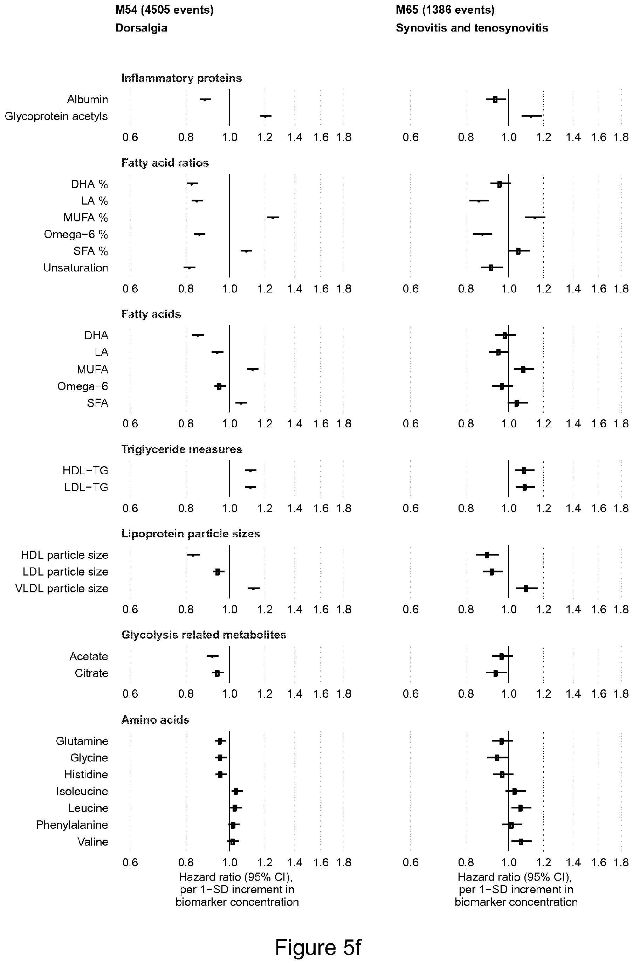

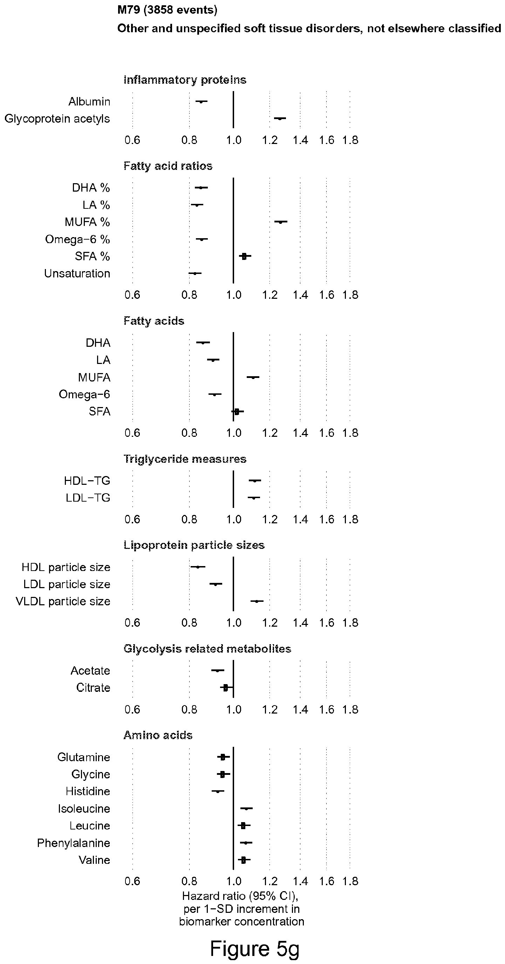

**Figures 5a-g** show the hazard ratios for the 27 blood biomarkers with future onset of each of the 13 specific musculoskeletal and/or connective tissue diseases (defined by ICD-10 3-character diagnosis codes) studied here. The hazard ratios are shown in absolute concentrations, scaled to the standard deviation of each biomarker. The results are based on statistical analyses of over 115 000 individuals from the UK Biobank; the number of individuals who developed the specific disease during approximately 10 years of follow-up is indicated on the top of each plot. Filled circles denote that the P-value for association was P<0.0001 (corresponding to multiple testing correction), and open circles denote that the P-value for association was P≥0.0001. The analyses were adjusted for age, sex, and UK Biobank assessment centre using Cox proportional-hazard regression models.

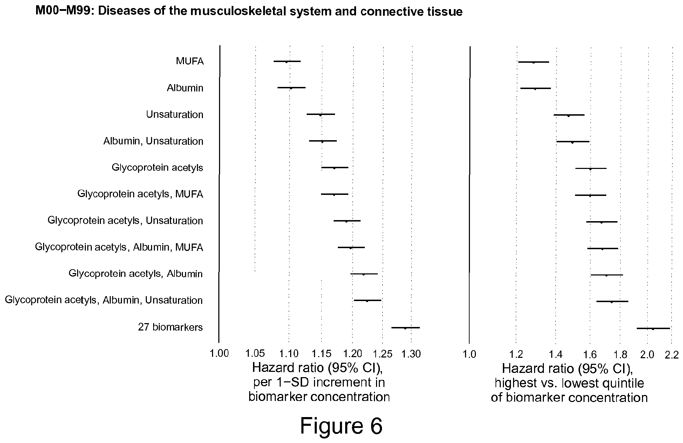

**Figure 6** shows examples of stronger association results with Any Musculoskeletal and/or Connective Tissue Disease when two or more biomarkers are combined. The hazard ratios with the future risk of Any Musculoskeletal and/or Connective Tissue Disease (composite endpoint of ICD-10 codes M00-M99) are shown for selected combinations of pairs of biomarkers, and examples of multi-biomarker scores. The results were similar with many other combinations, in particular inclusion of different fatty acid measures in addition to albumin and glycoprotein acetyls. The multi-biomarker scores are combined in the form of

$$\sum_i [\![ \beta_i * c_i ]\!] + \beta_0;$$

where $i$ is the index of summation over individual biomarkers, $\beta_i$ is the weighted coefficient attributed to biomarker $i$, $c_i$ is the blood concentration of biomarker $i$ and $\beta_0$ is an intercept term. $\beta_i$ multipliers are defined according to the multivariate association magnitude with the risk for Any Musculoskeletal and/or Connective Tissue Disease, examined in the statistical analyses of the UK Biobank study for the respective combination of biomarkers. The enhancements in association magnitudes were similar for the 13 specific types of musculoskeletal and/or connective tissue diseases listed in Table 1 as those shown here for Any Musculoskeletal and/or Connective Tissue Disease.

**Illustrations of intended use: multi-biomarker scores for risk prediction of musculoskeletal and/or connective tissue disease**

For illustration of intended applications related to the prediction of a musculoskeletal and/or connective tissue disease, further epidemiological analyses are illustrated below. These applications are exemplified for the prediction of the risk for rheumatoid arthritis, other arthritis, gout, osteoarthritis, joint disorders, spondylosis and/or soft tissue disorders. Similar results apply to the other musculoskeletal and/or connective tissue diseases listed in Table 1. Results are shown for a multi-biomarker score combining the 27 biomarkers featured in Figures 1-6. Similar results, albeit slightly weaker, are obtained with combinations of only two or three individual biomarkers.

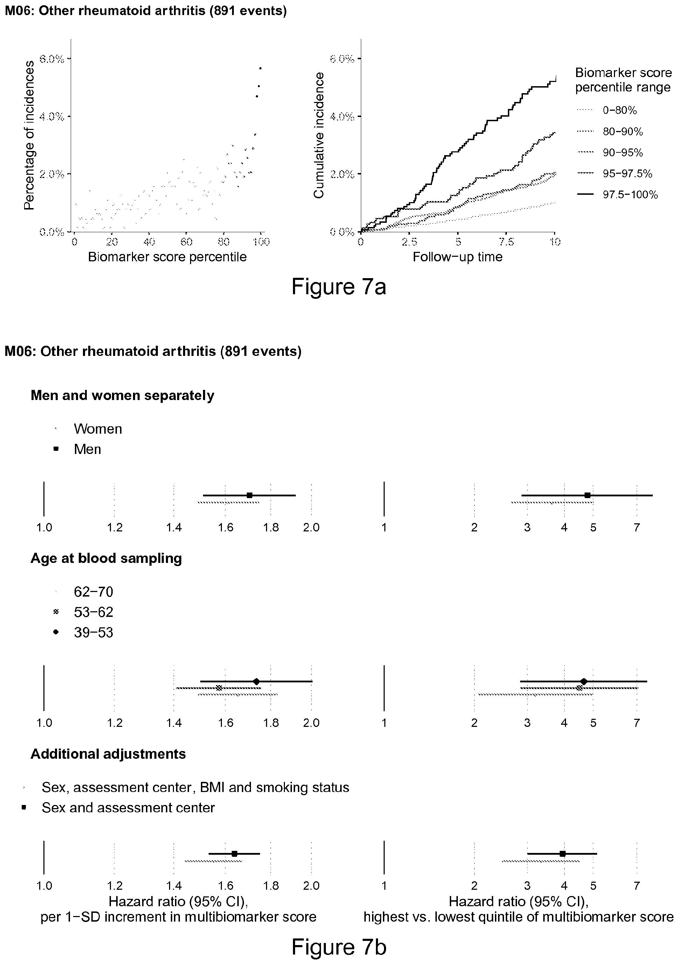

**Figure 7a** shows the increase in the risk for rheumatoid arthritis (ICD-10 code M06) along with increasing levels of a multi-biomarker score composed of the weighted sum of 27 biomarkers. On the left-hand side, the risk increase is plotted in the form of gradient percentile plots, showing the proportion of individuals who developed rheumatoid arthritis during follow-up when binning individuals into the percentiles of the biomarker levels. Each dot corresponds to approximately 500 individuals. In the Kaplan-Meier plots on the right-hand side, the cumulative risk for rheumatoid arthritis during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included in derivation of the multi-biomarker score (n = 63 136 individuals).

**Figure 7b** shows the hazard ratio of the same multi-biomarker score with the future onset of rheumatoid arthritis (ICD-10 code M06) when accounting for relevant risk factor characteristics of the study participants. The first panel demonstrates that the risk prediction works effectively for men and women, with slightly stronger results for women. The second panel shows that risk prediction also works for people at different ages at the time of blood sampling, with stronger results for younger individuals. The third panel shows that the magnitude of the hazard ratio is only modestly attenuated when accounting for body mass index and smoking status in the statistical modelling.

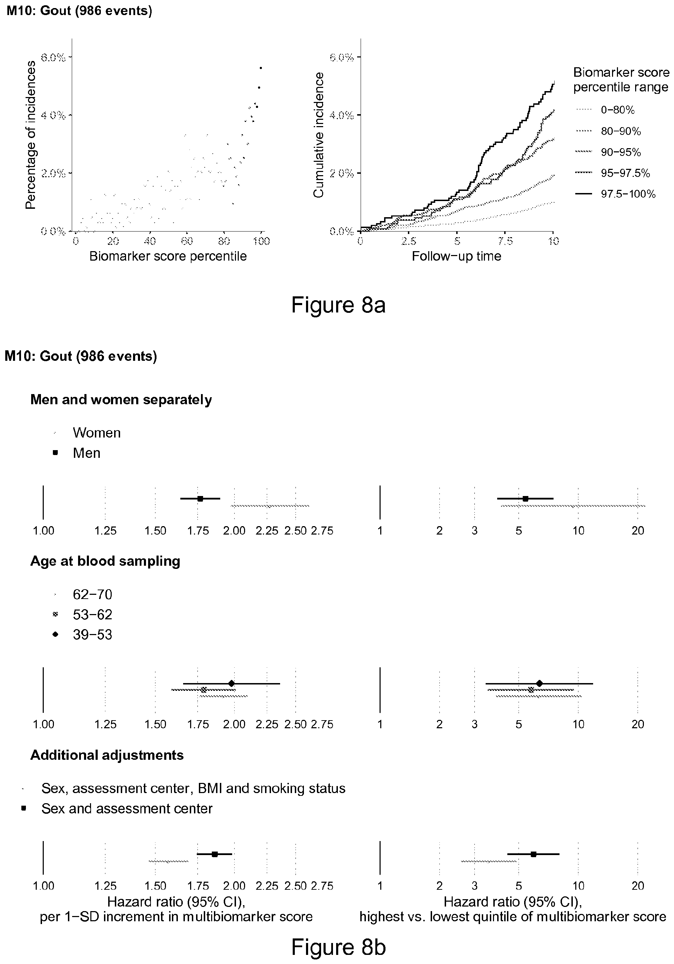

**Figure 8a** shows the increase in the risk for gout (ICD-10 code M10) along with increasing levels of a multi-biomarker score composed of the weighted sum of 27 biomarkers. On the left-hand side, the risk increase is plotted in the form of gradient percentile plots, showing the proportion of individuals who developed gout during follow-up when binning individuals into the percentiles of the biomarker levels. Each dot corresponds to approximately 500 individuals. In the Kaplan-Meier plots on the right-hand side, the cumulative risk for gout during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included in derivation of the multi-biomarker score (n = 63 416 individuals).

**Figure 8b** shows the hazard ratio of the same multi-biomarker score with the future onset of gout (ICD-10 code M10) when accounting for relevant risk factor characteristics of the study participants. The first panel demonstrates that the risk prediction works effectively for men and women, with slightly stronger results for women. The second panel shows that risk prediction also works effectively for people at different ages at the time of blood sampling. The third panel shows that the magnitude of the hazard ratio is only modestly attenuated when accounting for body mass index and smoking status in the statistical modelling.

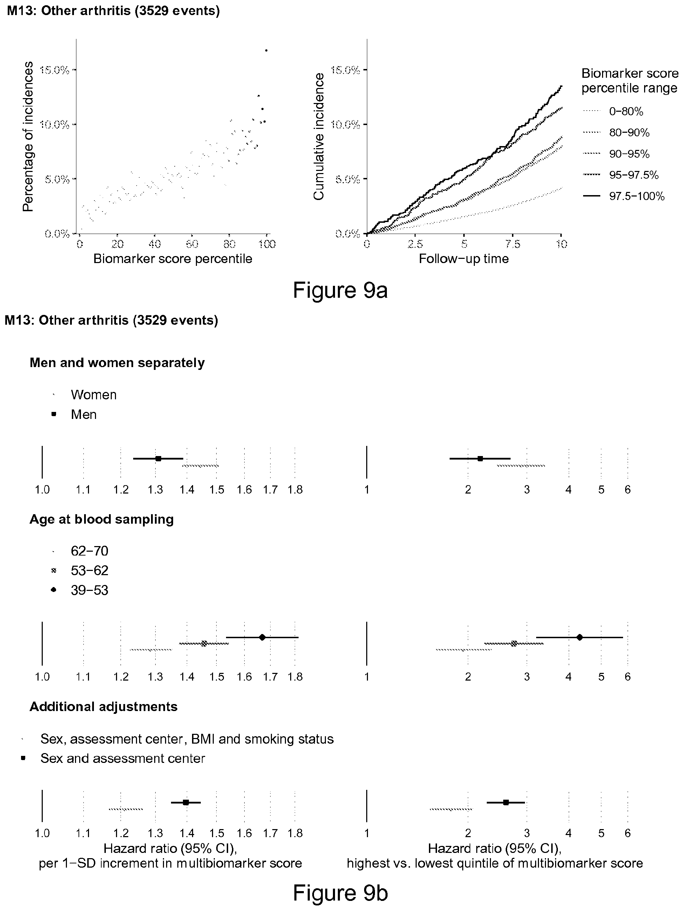

**Figure 9a** shows the increase in the risk for other arthritis (ICD-10 code M13) along with increasing levels of a multi-biomarker score composed of the weighted sum of 27 biomarkers. On the left-hand side, the risk increase is plotted in the form of gradient percentile plots, showing the proportion of individuals who developed arthritis during follow-up when binning individuals into the percentiles of the biomarker levels. Each dot corresponds to approximately 500 individuals. In the Kaplan-Meier plots on the right-hand side, the cumulative risk for arthritis during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included in derivation of the multi-biomarker score (n = 62 703 individuals).

**Figure 9b** shows the hazard ratio of the same multi-biomarker score with the future onset of arthritis (ICD-10 code M13) when accounting for relevant risk factor characteristics of the study participants. The first panel demonstrates that the risk prediction works effectively for men and women, with slightly stronger results for women. The second panel shows that risk prediction also works for people at different ages at the time of blood sampling, with stronger results for younger individuals. The third panel shows that the magnitude of the hazard ratio is only modestly attenuated when accounting for body mass index and smoking status in the statistical modelling.

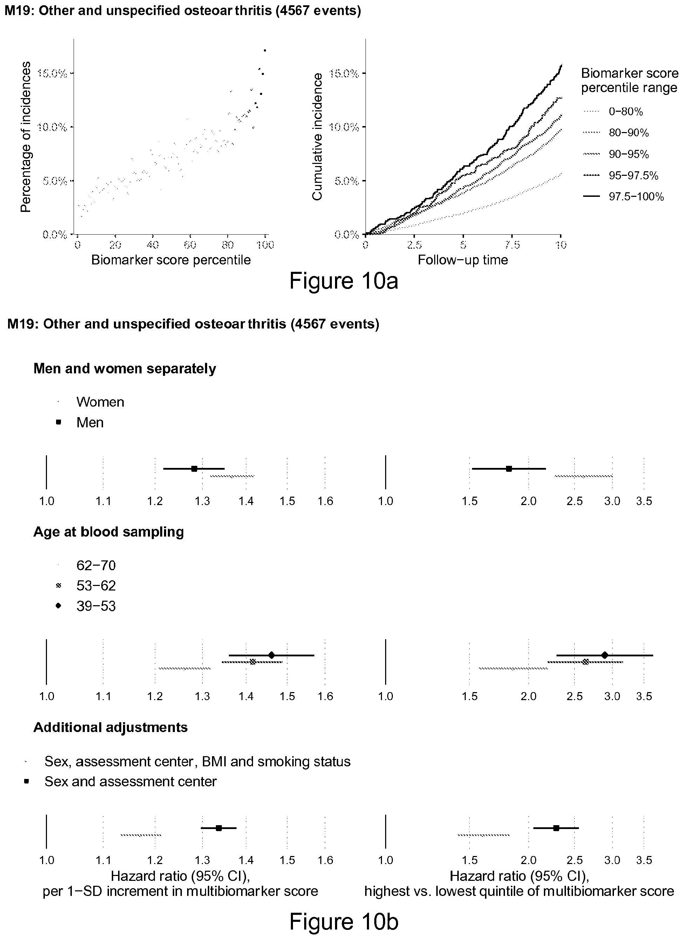

**Figure 10a** shows the increase in the risk for osteoarthritis (ICD-10 code M19) along with increasing levels of a multi-biomarker score composed of the weighted sum of 27 biomarkers. On the left-hand side, the risk increase is plotted in the form of gradient percentile plots, showing the proportion of individuals who developed osteoarthritis during follow-up when binning individuals into the percentiles of the biomarker levels. Each dot corresponds to approximately 500 individuals. In the Kaplan-Meier plots on the right-hand side, the cumulative risk for osteoarthritis during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included in derivation of the multi-biomarker score (n = 62 175 individuals).

**Figure 10b** shows the hazard ratio of the same multi-biomarker score with the future onset of osteoarthritis (ICD-10 code M19) when accounting for relevant risk factor characteristics of the study participants. The first panel demonstrates that the risk prediction works effectively for men and women, with slightly stronger results for women. The second panel shows that risk prediction also works for people at different ages at the time of blood sampling, with stronger results for younger individuals. The third panel shows that the magnitude of the hazard ratio is only modestly attenuated when accounting for body mass index and smoking status in the statistical modelling.

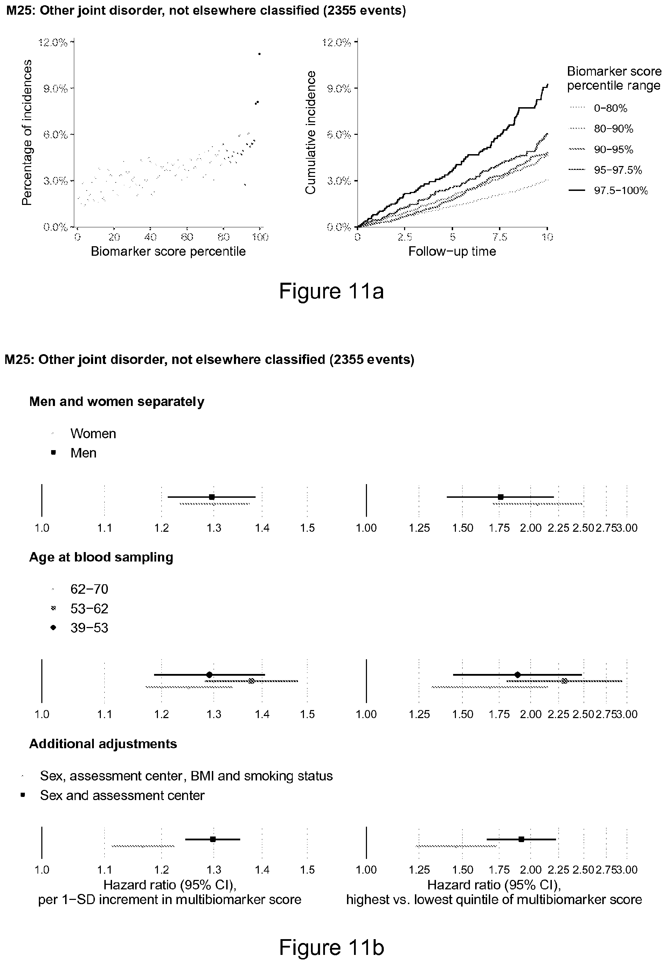

**Figure 11a** shows the increase in the risk for joint disorder (ICD-10 code M25) along with increasing levels of a multi-biomarker score composed of the weighted sum of 27 biomarkers. On the left-hand side, the risk increase is plotted in the form of gradient percentile plots, showing the proportion of individuals who developed joint disorder during follow-up when binning individuals into the percentiles of the biomarker levels. Each dot corresponds to approximately 500 individuals. In the Kaplan-Meier plots on the right-hand side, the cumulative risk for joint disorder during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included in derivation of the multi-biomarker score (n = 61 721 individuals).

**Figure 11b** shows the hazard ratio of the same multi-biomarker score with the future onset of joint disorder (ICD-10 code M25) when accounting for relevant risk factor characteristics of the study participants. The first two panels demonstrate that the risk prediction works effectively for men and women, and for people of different ages at the time of blood sampling. The third panel shows that the magnitude of the hazard ratio is only modestly attenuated when accounting for body mass index and smoking status in the statistical modelling.

**Figure 12a** shows the increase in the risk for spondylosis (ICD-10 code M47) along with increasing levels of a multi-biomarker score composed of the weighted sum of 27 biomarkers. On the left-hand side, the risk increase is plotted in the form of gradient percentile plots, showing the proportion of individuals who developed spondylosis during follow-up when binning individuals into the percentiles of the biomarker levels. Each dot corresponds to approximately 500 individuals. In the Kaplan-Meier plots on the right-hand side, the cumulative risk for spondylosis during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included in derivation of the multi-biomarker score (n = 62 850 individuals).

**Figure 12b** shows the hazard ratio of the same multi-biomarker score with the future onset of spondylosis (ICD-10 code M47) when accounting for relevant risk factor characteristics of the study participants. The first panel demonstrates that the risk prediction works effectively for men and women, with slightly stronger results for women. The second panel shows that risk prediction also works for people at different ages at the time of blood sampling, with stronger results for younger individuals. The third panel shows that the magnitude of the hazard ratio is only modestly attenuated when accounting for body mass index and smoking status in the statistical modelling.

**Figure 13a** shows the increase in the risk for soft tissue disorders (ICD-10 code M79) along with increasing levels of a multi-biomarker score composed of the weighted sum of 27 biomarkers. On the left-hand side, the risk increase is plotted in the form of gradient percentile plots, showing the proportion of individuals who developed soft tissue disorders during follow-up when binning individuals into the percentiles of the biomarker levels. Each dot corresponds to approximately 500 individuals. In the Kaplan-Meier plots on the right-hand side, the cumulative risk for soft tissue disorders during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included in derivation of the multi-biomarker score (n = 61 870 individuals).

**Figure 13b** shows the hazard ratio of the same multi-biomarker score with the future onset of soft tissue disorders (ICD-10 code M79) when accounting for relevant risk factor characteristics of the study participants. The first panel demonstrates that the risk prediction works effectively for men and women, with slightly stronger results for women. The second panel shows that risk prediction also works for people at different ages at the time of blood sampling, with stronger results for younger individuals. The third panel shows that the magnitude of the hazard ratio is only modestly attenuated when accounting for body mass index and smoking status in the statistical modelling.

It is obvious to a person skilled in the art that with the advancement of technology, the basic idea may be implemented in various ways. The embodiments are thus not limited to the examples described above; instead they may vary within the scope of the claims.

The embodiments described hereinbefore may be used in any combination with each other. Several of the embodiments may be combined together to form a further embodiment. A method disclosed herein may comprise at least one of the embodiments described hereinbefore. It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item refers to one or more of those items. The term "comprising" is used in this specification to mean including the feature(s) or act(s) followed thereafter, without excluding the presence of one or more additional features or acts.

**Claims**

1.   A method for determining whether a subject is at risk of developing a musculoskeletal disease;

   wherein the method comprises determining in a biological sample obtained from the subject a quantitative value of at least one biomarker of the following in the biological sample:

   - glycoprotein acetyls,
   - albumin,
   - a ratio of docosahexaenoic acid to total fatty acids,
   - a ratio of linoleic acid to total fatty acids
   - a ratio of monounsaturated fatty acids and/or of oleic acid to total fatty acids,
   - a ratio of omega-6 fatty acids to total fatty acids,
   - a ratio of saturated fatty acids to total fatty acids,
   - fatty acid degree of unsaturation,
   - docosahexaenoic acid,
   - linoleic acid,
   - monounsaturated fatty acids and/or oleic acid,
   - omega-6 fatty acids,
   - saturated fatty acids,
   - triglycerides in high-density lipoprotein (HDL),
   - triglycerides in low-density lipoprotein (LDL),
   - high-density lipoprotein (HDL) particle size,
   - low-density lipoprotein (LDL) particle size,
   - very-low-density lipoprotein (VLDL) particle size,

- acetate,
- citrate,
- glutamine,
- glycine,
- histidine,
- isoleucine,
- leucine,
- phenylalanine,
- valine; and

comparing the quantitative value(s) of the at least one biomarker to a control sample or to a control value; wherein an increase or a decrease in the quantitative value (s) of the at least one biomarker, when compared to the control sample or to the control value, is/are indicative of the subject having an increased risk of developing a musculoskeletal disease; wherein the at least one biomarker comprises or is glycoprotein acetyls; and wherein the musculoskeletal disease comprises or is osteoarthritis.

2. The method according to claim 1, wherein the method comprises determining in the biological sample quantitative values of a plurality of the biomarkers, such as two, three, four, five or more biomarkers.

3. The method according to claim 1 or 2, wherein the method comprises determining in the biological sample obtained from the subject a quantitative value of the following biomarkers:

   - glycoprotein acetyls;
   - albumin; and
   comparing the quantitative value(s) of the biomarkers to a control sample or to a control value(s); wherein an increase or a decrease in the quantitative value(s) of the biomarkers, when compared to the control sample or to the control value, is/are indicative of the subject having an increased risk of developing a musculoskeletal disease.

4. The method according to any one of claims 1 - 3, wherein the method comprises determining in the biological sample obtained from the subject a quantitative value of the following biomarkers:

   - glycoprotein acetyls,
   - at least one fatty acid measure (s) of the following: ratio of docosahexaenoic acid to total fatty acids, docosahexaenoic acid, ratio of linoleic acid to total fatty acids, linoleic acid, ratio of monounsaturated fatty acids and/or of oleic acid to total fatty acids, ratio of omega-6 fatty acids to total fatty acids, omega-6 fatty acids, ratio of saturated fatty acids to total fatty acids, saturated fatty acids, fatty acid degree of unsaturation; and comparing the quantitative value(s) of the biomarkers to a control sample or to a control value(s); wherein an increase or a decrease in the quantitative value(s) of the biomarkers, when compared to the control sample or to the control value, is/are indicative of the subject having an increased risk of developing a musculoskeletal disease.

5. The method according to any one of claims 1 - 4, wherein the musculoskeletal disease comprises or is polyosteoarthritis (M15); osteoarthritis of knee (M17); and/or other and unspecified osteoarthritis (M19).

6. The method according to any one of claims 1 - 5, wherein the quantitative value of the at least one biomarker is/are measured using nuclear magnetic resonance spectroscopy.

7. The method according to any one of claims 1 - 6, wherein the method further comprises determining whether the subject is at risk of developing a musculoskeletal disease using a risk score, hazard ratio, odds ratio, and/or predicted absolute risk or relative risk calculated on the basis of the quantitative value(s) of the at least one biomarker or of the plurality of the biomarkers.

8. The method according to claim 7, wherein the risk score, hazard ratio, odds ratio, and/or predicted relative risk and/or absolute risk is calculated on the basis of at least one further measure, such as a characteristic of the subject.

9. The method according to claim 8, wherein the characteristic of the subject includes one or more of age, height, weight, body mass index, race or ethnic group, smoking, and/or family history of musculoskeletal and/or connective tissue diseases.

10. The method according to any one of claims 1 - 9, wherein the method comprises determining in the biological sample obtained from the subject a quantitative value of the following biomarkers:

- glycoprotein acetyls,
- albumin,
- a ratio of docosahexaenoic acid to total fatty acids,
- a ratio of linoleic acid to total fatty acids,
- a ratio of monounsaturated fatty acids and/or of oleic acid to total fatty acids,
- a ratio of omega-6 fatty acids to total fatty acids,
- a ratio of saturated fatty acids to total fatty acids,
- fatty acid degree of unsaturation,
- docosahexaenoic acid,
- linoleic acid,
- monounsaturated fatty acids and/or oleic acid,
- omega-6 fatty acids,
- saturated fatty acids,
- triglycerides in high-density lipoprotein (HDL),
- triglycerides in low-density lipoprotein (LDL),
- high-density lipoprotein (HDL) particle size,
- low-density lipoprotein (LDL) particle size,
- very-low-density lipoprotein (VLDL) particle size,
- acetate,
- citrate,
- glutamine,
- glycine,
- histidine,
- isoleucine,
- leucine,
- phenylalanine,
- valine; and
comparing the quantitative value(s) of the biomarkers to a control sample or to a control value(s);
wherein an increase or a decrease in the quantitative value(s) of the biomarkers, when compared to the control sample or to the control value, is/are indicative of the subject having an increased risk of developing the musculoskeletal disease.

**Patentansprüche**

1. Verfahren zur Bestimmung des Risikos, dass ein Subjekt eine Muskel-Skelett-Erkrankung entwickelt;

wobei das Verfahren die Bestimmung eines quantitativen Wertes von mindestens einem der folgenden Biomarker in einer biologischen Probe, die von der Person erhalten wurde, umfasst:

- Glykoprotein-Acetyls,
- Albumin,
- das Verhältnis von Docosahexaensäure zu den Gesamtfettsäuren,
- das Verhältnis von Linolsäure zu den Gesamtfettsäuren
- ein Verhältnis von einfach ungesättigten Fettsäuren und/oder Ölsäure zu den Gesamtfettsäuren,
- ein Verhältnis von Omega-6-Fettsäuren zu den Gesamtfettsäuren,
- das Verhältnis von gesättigten Fettsäuren zu den Gesamtfettsäuren,
- Grad der Ungesättigtheit der Fettsäuren,
- Docosahexaensäure,
- Linolsäure,
- einfach ungesättigte Fettsäuren und/oder Ölsäure,
- Omega-6-Fettsäuren,

- gesättigte Fettsäuren,
- Triglyceride im High-Density-Lipoprotein (HDL),
- Triglyceride im Low-Density-Lipoprotein (LDL),
- die Partikelgröße von Lipoproteinen hoher Dichte (HDL),
- Low-Density-Lipoprotein (LDL)-Partikelgröße,
- Partikelgröße von Lipoproteinen sehr niedriger Dichte (VLDL),
- Acetat,
- Zitrat,
- Glutamin,
- Glycin,
- Histidin,
- Isoleucin,
- Leucin,
- Phenylalanin,
- Valin; und

Vergleich des quantitativen Wertes/der quantitativen Werte des mindestens einen Biomarkers mit einer Kontrollprobe oder mit einem Kontrollwert;
wobei eine Zunahme oder eine Abnahme des quantitativen Wertes/der quantitativen Werte des mindestens einen Biomarkers im Vergleich zur Kontrollprobe oder zum Kontrollwert ein Anzeichen dafür ist/sind, dass das Subjekt ein erhöhtes Risiko hat, eine Muskel-Skelett-Erkrankung zu entwickeln;
wobei der mindestens eine Biomarker Glykoproteinacetyls umfasst oder ist; und wobei die Muskel- und Skeletterkrankung Osteoarthritis umfasst oder eine solche ist.

2. Verfahren nach Anspruch 1, wobei das Verfahren die Bestimmung quantitativer Werte einer Vielzahl von Biomarkern, wie zwei, drei, vier, fünf oder mehr Biomarker, in der biologischen Probe umfasst.

3. Verfahren nach Anspruch 1 oder 2, wobei das Verfahren die Bestimmung eines quantitativen Wertes der folgenden Biomarker in der von der Person erhaltenen biologischen Probe umfasst:

- Glykoprotein-Acetyls;
- Albumin; und
Vergleich des quantitativen Wertes/der quantitativen Werte der Biomarker mit einer Kontrollprobe oder mit einem Kontrollwert/mehreren Kontrollwerten;
wobei eine Zunahme oder eine Abnahme des quantitativen Wertes/der quantitativen Werte der Biomarker im Vergleich zur Kontrollprobe oder zum Kontrollwert ein Anzeichen dafür ist/sind, dass das Subjekt ein erhöhtes Risiko hat, eine Muskel-Skelett-Erkrankung zu entwickeln.

4. Verfahren nach einem der Ansprüche 1 bis 3, wobei das Verfahren die Bestimmung eines quantitativen Wertes der folgenden Biomarker in der von der Person erhaltenen biologischen Probe umfasst:

- Glykoprotein-Acetyle,
- mindestens eine der folgenden Fettsäuremessungen: Verhältnis der Docosahexaensäure zu den Gesamtfettsäuren, Docosahexaensäure, Verhältnis der Linolsäure zu den Gesamtfettsäuren, Linolsäure, Verhältnis der einfach ungesättigten Fettsäuren und/oder der Ölsäure zu den Gesamtfettsäuren, Verhältnis der Omega-6-Fettsäuren zu den Gesamtfettsäuren, Omega-6-Fettsäuren, Verhältnis der gesättigten Fettsäuren zu den Gesamtfettsäuren, gesättigte Fettsäuren, Ungesättigtheitsgrad der Fettsäuren; und
Vergleich des quantitativen Wertes/der quantitativen Werte der Biomarker mit einer Kontrollprobe oder mit einem Kontrollwert/mehreren Kontrollwerten;
wobei eine Zunahme oder eine Abnahme des quantitativen Wertes/der quantitativen Werte der Biomarker im Vergleich zur Kontrollprobe oder zum Kontrollwert ein Anzeichen dafür ist/sind, dass das Subjekt ein erhöhtes Risiko hat, eine Muskel-Skelett-Erkrankung zu entwickeln.

5. Verfahren nach einem der Ansprüche 1 bis 4, wobei die Muskel-Skelett-Erkrankung Polyosteoarthritis (M15), Knie-Osteoarthritis (M17) und/oder andere und nicht spezifizierte Osteoarthritis (M19) umfasst oder ist.

6. Verfahren nach einem der Ansprüche 1 bis 5, wobei der quantitative Wert des mindestens einen Biomarkers unter Verwendung der kernmagnetischen Resonanzspektroskopie gemessen wird/werden.

**7.** Verfahren nach einem der Ansprüche 1 bis 6, wobei das Verfahren ferner die Bestimmung umfasst, ob das Subjekt ein Risiko hat, eine Muskel-Skelett-Erkrankung zu entwickeln, unter Verwendung eines Risiko-Scores, eines Hazard-Ratios, eines Odds-Ratios und/oder eines vorhergesagten absoluten Risikos oder eines relativen Risikos, das auf der Grundlage des quantitativen Wertes/der quantitativen Werte des mindestens einen Biomarkers oder der Vielzahl der Biomarker berechnet wird.

**8.** Verfahren nach Anspruch 7, wobei der Risikoscore, das Hazard Ratio, das Odds Ratio und/oder das vorhergesagte relative Risiko und/oder das absolute Risiko auf der Grundlage mindestens eines weiteren Maßes, wie z. B. einer Eigenschaft des Probanden, berechnet wird.

**9.** Verfahren nach Anspruch 8, wobei das Merkmal des Probanden eines oder mehrere der folgenden Merkmale umfasst: Alter, Größe, Gewicht, Body-Mass-Index, Rasse oder ethnische Gruppe, Rauchen und/oder Familienanamnese von Erkrankungen des Bewegungsapparats und/oder des Bindegewebes.

**10.** Verfahren nach einem der Ansprüche 1 bis 9, wobei das Verfahren die Bestimmung eines quantitativen Wertes der folgenden Biomarker in der von der Person erhaltenen biologischen Probe umfasst:

- Glykoprotein-Acetyle,
- Albumin,
- das Verhältnis von Docosahexaensäure zu den Gesamtfettsäuren,
- das Verhältnis von Linolsäure zu den Gesamtfettsäuren,
- ein Verhältnis von einfach ungesättigten Fettsäuren und/oder Ölsäure zu den Gesamtfettsäuren,
- ein Verhältnis von Omega-6-Fettsäuren zu den Gesamtfettsäuren,
- das Verhältnis von gesättigten Fettsäuren zu den Gesamtfettsäuren,
- Grad der Ungesättigtheit der Fettsäuren,
- Docosahexaensäure,
- Linolsäure,
- einfach ungesättigte Fettsäuren und/oder Ölsäure,
- Omega-6-Fettsäuren,
- gesättigte Fettsäuren,
- Triglyceride im High-Density-Lipoprotein (HDL),
- Triglyceride im Low-Density-Lipoprotein (LDL),
- die Partikelgröße von Lipoproteinen hoher Dichte (HDL),
- Low-Density-Lipoprotein (LDL)-Partikelgröße,
- Partikelgröße von Lipoproteinen sehr geringer Dichte (VLDL),
- Acetat,
- Zitrat,
- Glutamin,
- Glycin,
- Histidin,
- Isoleucin,
- Leucin,
- Phenylalanin,
- Valin; und

Vergleich des quantitativen Wertes/der quantitativen Werte der Biomarker mit einer Kontrollprobe oder mit einem Kontrollwert/mehreren Kontrollwerten;
wobei eine Zunahme oder eine Abnahme des quantitativen Wertes/der quantitativen Werte der Biomarker im Vergleich zur Kontrollprobe oder zum Kontrollwert ein Anzeichen dafür ist/sind, dass das Subjekt ein erhöhtes Risiko für die Entwicklung der Muskel-Skelett-Erkrankung hat.

**Revendications**

**1.** Procédé pour déterminer si un sujet risque de développer une maladie musculosquelettique ;

lequel procédé comprend la détermination, dans un échantillon biologique obtenu auprès du sujet, d'une valeur quantitative d'au moins un marqueur biologique de ce qui suit dans l'échantillon biologique ;

- les acétyl-glycoprotéines,
- l'albumine,
- le rapport de l'acide docosahexénoïque aux acides gras totaux,
- le rapport de l'acide linoléique aux acides gras totaux,
- le rapport des acides gras monoinsaturés et/ou de l'acide oléique aux acides gras totaux,
- le rapport des acides gras oméga-6 aux acides gras totaux,
- le rapport des acides gras saturés aux acides gras totaux,
- le degré d'insaturation des acides gras,
- l'acide docosahexénoïque,
- l'acide linoléique,
- les acides gras monoinsaturés et/ou l'acide oléique,
- les acides gras oméga-6,
- les acides gras saturés,
- les triglycérides dans les lipoprotéines haute densité (HDL),
- les triglycérides dans les lipoprotéines basse densité (LDL),
- la taille des particules de lipoprotéines haute densité (HDL),
- la taille des particules de lipoprotéines basse densité (LDL),
- la taille des particules de lipoprotéines très basse densité (VLDL),
- l'acétate,
- le citrate,
- la glutamine,
- la glycine,
- l'histidine,
- l'isoleucine,
- la leucine,
- la phénylalanine,
- la valine ; et

la comparaison de la ou des valeurs quantitatives de l'au moins un marqueur biologique avec un échantillon témoin ou avec une valeur témoin ;
dans lequel une augmentation ou une diminution de la ou des valeurs quantitatives de l'au moins un marqueur biologique, en comparaison avec l'échantillon témoin ou la valeur témoin, indique que le sujet présente un plus grand risque de développer une maladie musculosquelettique ;
dans lequel l'au moins un marqueur biologique comprend ou consiste en les acétyl-glycoprotéines ; et
dans lequel la maladie musculosquelettique comprend ou consiste en l'arthrose.

2. Procédé selon la revendication 1, lequel procédé comprend la détermination, dans l'échantillon biologique, de valeurs quantitatives d'une pluralité des marqueurs biologiques, telle que deux, trois, quatre, cinq ou plus de cinq marqueurs biologiques.

3. Procédé selon la revendication 1 ou 2, lequel procédé comprend la détermination, dans l'échantillon biologique obtenu auprès du sujet, d'une valeur quantitative des marqueurs biologiques suivants :

- les acétyl-glycoprotéines ;
- l'albumine ; et
la comparaison de la ou des valeurs quantitatives des marqueurs biologiques avec un échantillon témoin ou avec une ou plusieurs valeurs témoins ;
dans lequel une augmentation ou une diminution de la ou des valeurs quantitatives des marqueurs biologiques, en comparaison avec l'échantillon témoin ou la valeur témoin, indique que le sujet présente un plus grand risque de développer une maladie musculosquelettique.

4. Procédé selon l'une quelconque des revendications 1 à 3, lequel procédé comprend la détermination, dans l'échantillon biologique obtenu auprès du sujet, d'une valeur quantitative des marqueurs biologiques suivants :

- les acétyl-glycoprotéines ;
- au moins une mesure d'acide gras parmi les suivantes : le rapport de l'acide docosahexénoïque aux acides gras totaux, l'acide docosahexénoïque, le rapport de l'acide linoléique aux acides gras totaux, l'acide linoléique, le rapport des acides gras monoinsaturés et/ou de l'acide oléique aux acides gras totaux, le rapport des acides gras oméga-6 aux acides gras totaux, les acides gras oméga-6, le rapport des acides gras saturés aux acides gras totaux, les acides gras saturés, le degré d'insaturation des acides gras ; et

la comparaison de la ou des valeurs quantitatives des marqueurs biologiques avec un échantillon témoin ou avec une ou plusieurs valeurs témoins ;

dans lequel une augmentation ou une diminution de la ou des valeurs quantitatives des marqueurs biologiques, en comparaison avec l'échantillon témoin ou la valeur témoin, indique que le sujet présente un plus grand risque de développer une maladie musculosquelettique.

5. Procédé selon l'une quelconque des revendications 1 à 4, dans lequel la maladie musculosquelettique comprend ou est la polyarthrose (M15) ; l'arthrose du genou (M17) ; et/ou une autre arthrose ou une arthrose non spécifiée (M19).

6. Procédé selon l'une quelconque des revendications 1 à 5, dans lequel la valeur quantitative de l'au moins un marqueur biologique est mesurée au moyen d'une spectroscopie à résonance magnétique nucléaire.

7. Procédé selon l'une quelconque des revendications 1 à 6, lequel procédé comprend en outre la détermination que le sujet risque ou non de développer une maladie musculosquelettique utilisant le score de risque, le rapport de risque, le rapport de cotes, et/ou le risque absolu ou risque relatif prédit calculé sur la base de la ou des valeurs quantitatives de l'au moins un marqueur biologique ou de la pluralité de marqueurs biologiques.

8. Procédé selon la revendication 7, dans lequel le score de risque, le rapport de risque, le rapport de cotes, et/ou le risque absolu et/ou risque relatif prédit sont calculés sur la base d'au moins une autre mesure, telle qu'une caractéristique du sujet.

9. Procédé selon la revendication 8, dans lequel la caractéristique du sujet comprend un ou plusieurs parmi l'âge, la taille, le poids, l'indice de masse corporelle, la race ou le groupe ethnique, le tabagisme, et/ou l'historique familial de maladies musculosquelettiques et/ou du tissu conjonctif.

10. Procédé selon l'une quelconque des revendications 1 à 9, lequel procédé comprend la détermination, dans l'échantillon biologique obtenu auprès du sujet, d'une valeur quantitative des marqueurs biologiques suivants :

- les acétyl-glycoprotéines ;
- l'albumine,
- le rapport de l'acide docosahexénoïque aux acides gras totaux,
- le rapport de l'acide linoléique aux acides gras totaux,
- le rapport des acides gras monoinsaturés et/ou de l'acide oléique aux acides gras totaux,
- le rapport des acides gras oméga-6 aux acides gras totaux,
- le rapport des acides gras saturés aux acides gras totaux,
- le degré d'insaturation des acides gras,
- l'acide docosahexénoïque,
- l'acide linoléique,
- les acides gras monoinsaturés et/ou l'acide oléique,
- les acides gras oméga-6,
- les acides gras saturés,
- les triglycérides dans les lipoprotéines haute densité (HDL),
- les triglycérides dans les lipoprotéines basse densité (LDL),
- la taille des particules de lipoprotéines haute densité (HDL),
- la taille des particules de lipoprotéines basse densité (LDL),
- la taille des particules de lipoprotéines très basse densité (VLDL),
- l'acétate,
- le citrate,
- la glutamine,
- la glycine,
- l'histidine,
- l'isoleucine,
- la leucine,
- la phénylalanine,
- la valine ; et

la comparaison de la ou des valeurs quantitatives des marqueurs biologiques avec un échantillon témoin ou avec une ou plusieurs valeurs témoins ;

dans lequel une augmentation ou une diminution de la ou des valeurs quantitatives des marqueurs biologiques, en comparaison avec l'échantillon témoin ou la valeur témoin, indique que le sujet présente un plus grand risque de développer une maladie musculosquelettique.

**M00−M99: Diseases of the musculoskeletal system and connective tissue**
**22 986 events**

Figure 1a

Figure 1b

Figure 2a

Figure 2b

Figure 3a

Figure 3b

Figure 4a

Figure 4b

Figure 4c

**M70−M79: (7478 events)**
**Other soft tissue disorders**

Inflammatory proteins

Albumin
Glycoprotein acetyls

0.8   0.9   1.0   1.1   1.2   1.3   1.4

Fatty acid ratios

DHA %
LA %
MUFA %
Omega−6 %
SFA %
Unsaturation

0.8   0.9   1.0   1.1   1.2   1.3   1.4

Fatty acids

DHA
LA
MUFA
Omega−6
SFA

0.8   0.9   1.0   1.1   1.2   1.3   1.4

Triglyceride measures

HDL−TG
LDL−TG

0.8   0.9   1.0   1.1   1.2   1.3   1.4

Lipoprotein particle sizes

HDL particle size
LDL particle size
VLDL particle size

0.8   0.9   1.0   1.1   1.2   1.3   1.4

Glycolysis related metabolites

Acetate
Citrate

0.8   0.9   1.0   1.1   1.2   1.3   1.4

Amino acids

Glutamine
Glycine
Histidine
Isoleucine
Leucine
Phenylalanine
Valine

0.8   0.9   1.0   1.1   1.2   1.3   1.4
Hazard ratio (95% CI),
per 1−SD increment in
biomarker concentration

Figure 4d

Figure 5a

**M13 (5802 events)**
**Other arthritis**

**M15 (2141 events)**
**Polyosteoarthritis**

Figure 5b

Figure 5c

**M25 (3911 events)**
Other joint disorder, not elsewhere classified

**M47 (3295 events)**
Spondylosis

Figure 5d

**M48 (1928 events)**
**Other spondylopathies**

**M51 (2260 events)**
**Thoracic, thoracolumbar, and lumbosacral intervertebral disc disorders**

Figure 5e

**M54 (4505 events)**
**Dorsalgia**

**M65 (1386 events)**
**Synovitis and tenosynovitis**

Inflammatory proteins

Albumin
Glycoprotein acetyls

0.6   0.8   1.0   1.2   1.4   1.6   1.8          0.6   0.8   1.0   1.2   1.4   1.6   1.8

Fatty acid ratios

DHA %
LA %
MUFA %
Omega−6 %
SFA %
Unsaturation

0.6   0.8   1.0   1.2   1.4   1.6   1.8          0.6   0.8   1.0   1.2   1.4   1.6   1.8

Fatty acids

DHA
LA
MUFA
Omega−6
SFA

0.6   0.8   1.0   1.2   1.4   1.6   1.8          0.6   0.8   1.0   1.2   1.4   1.6   1.8

Triglyceride measures

HDL−TG
LDL−TG

0.6   0.8   1.0   1.2   1.4   1.6   1.8          0.6   0.8   1.0   1.2   1.4   1.6   1.8

Lipoprotein particle sizes

HDL particle size
LDL particle size
VLDL particle size

0.6   0.8   1.0   1.2   1.4   1.6   1.8          0.6   0.8   1.0   1.2   1.4   1.6   1.8

Glycolysis related metabolites

Acetate
Citrate

0.6   0.8   1.0   1.2   1.4   1.6   1.8          0.6   0.8   1.0   1.2   1.4   1.6   1.8

Amino acids

Glutamine
Glycine
Histidine
Isoleucine
Leucine
Phenylalanine
Valine

0.6   0.8   1.0   1.2   1.4   1.6   1.8          0.6   0.8   1.0   1.2   1.4   1.6   1.8
Hazard ratio (95% CI),                          Hazard ratio (95% CI),
per 1−SD increment in                           per 1−SD increment in
biomarker concentration                         biomarker concentration

Figure 5f

**M79 (3858 events)**
**Other and unspecified soft tissue disorders, not elsewhere classified**

Hazard ratio (95% CI),
per 1−SD increment in
biomarker concentration

## Figure 5g

**M00–M99: Diseases of the musculoskeletal system and connective tissue**

Hazard ratio (95% CI),
per 1–SD increment in
biomarker concentration

Hazard ratio (95% CI),
highest vs. lowest quintile
of biomarker concentration

# Figure 6

**M06: Other rheumatoid arthritis (891 events)**

Figure 7a

**M06: Other rheumatoid arthritis (891 events)**

**Men and women separately**

· Women
■ Men

**Age at blood sampling**

· 62-70
⊛ 53-62
♦ 39-53

**Additional adjustments**

· Sex, assessment center, BMI and smoking status
■ Sex and assessment center

Hazard ratio (95% CI),
per 1-SD increment in multibiomarker score

Hazard ratio (95% CI),
highest vs. lowest quintile of multibiomarker score

Figure 7b

**M10: Gout (986 events)**

Figure 8a

**M10: Gout (986 events)**

**Men and women separately**

- Women
- Men

**Age at blood sampling**

- 62-70
- 53-62
- 39-53

**Additional adjustments**

- Sex, assessment center, BMI and smoking status
- Sex and assessment center

Hazard ratio (95% CI),
per 1-SD increment in multibiomarker score

Hazard ratio (95% CI),
highest vs. lowest quintile of multibiomarker score

Figure 8b

**M13: Other arthritis (3529 events)**

## Figure 9a

**M13: Other arthritis (3529 events)**

**Men and women separately**

- Women
- Men

**Age at blood sampling**

- 62–70
- 53–62
- 39–53

**Additional adjustments**

- Sex, assessment center, BMI and smoking status
- Sex and assessment center

Hazard ratio (95% CI),
per 1–SD increment in multibiomarker score

Hazard ratio (95% CI),
highest vs. lowest quintile of multibiomarker score

## Figure 9b

**M19: Other and unspecified osteoarthritis (4567 events)**

Figure 10a

**M19: Other and unspecified osteoarthritis (4567 events)**

Figure 10b

**M25: Other joint disorder, not elsewhere classified (2355 events)**

Figure 11a

**M25: Other joint disorder, not elsewhere classified (2355 events)**

**Men and women separately**

·  Women
■  Men

**Age at blood sampling**

·  62–70
※  53–62
◆  39–53

**Additional adjustments**

·  Sex, assessment center, BMI and smoking status
■  Sex and assessment center

Hazard ratio (95% CI),
per 1−SD increment in multibiomarker score

Hazard ratio (95% CI),
highest vs. lowest quintile of multibiomarker score

Figure 11b

**M47: Spondylosis (2079 events)**

Figure 12a

**M47: Spondylosis (2079 events)**

**Men and women separately**

· Women
■ Men

**Age at blood sampling**

· 62–70
※ 53–62
● 39–53

**Additional adjustments**

· Sex, assessment center, BMI and smoking status
■ Sex and assessment center

Hazard ratio (95% CI), per 1–SD increment in multibiomarker score

Hazard ratio (95% CI), highest vs. lowest quintile of multibiomarker score

Figure 12b

**M79: Other and unspecified soft tissue disorders, not elsewhere classified (2287 events)**

Figure 13a

**M79: Other and unspecified soft tissue disorders, not elsewhere classified (2287 events)**

Figure 13b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013053065 A1

**Non-patent literature cited in the description**

- **KETTUNEN et al.** *Nature Genetics,* 2012, vol. 44, 269-276
- **SOININEN et al.** *Circulation: Cardiovascular Genetics,* 2015, vol. 8, 212-206
- **RITCHIE et al.** *Cell Systems,* 2015, vol. 1 (4), 293-301
- **CONNELLY et al.** *J Transl Med.,* 2017, vol. 15 (1), 219
- **KETTUNEN et al.** *Circ Genom Precis Med.,* 2018, vol. 11, e002234
- **SOININEN et al.** *Analyst,* 2009, vol. 134, 1781-1785
- **WÜRTZ et al.** *American Journal of Epidemiology,* 2017, vol. 186 (9), 1084-1096
- **RITCHIE et al.** *Cell Syst.,* 2015, vol. 1 (4), 293-301
- **JULA et al.** *Arterioscler Thromb Vasc Biol,* 2005, vol. 25, 2152-2159
- **SUDLOW et al.** *PLoS Med.,* 2015, vol. 12 (3), e1001779
- **SOININEN et al.** *Circ Cardiovasc Genet,* 2015, vol. 8, 192-206
- **WÜRTZ et al.** *Am J Epidemiol,* 2017, vol. 186, 1084-1096